# EUROPEAN PATENT APPLICATION

(11) **EP 4 101 464 A1**
(43) Date of publication of application: **14.12.2022**
(21) Application number: 21750115.4
(22) Date of filing: 05.02.2021
(51) Int. Cl.: A61K 39/395, C12Q 1/68, A61P 35/00

(54) **USE OF ANTI-PD-1 ANTIBODY IN TREATMENT OF MALIGNANT TUMORS**

(30) Priority: 07.02.2020 CN 202010082208
(71) Applicant: Shanghai Junshi Biosciences Co., Ltd., Pilot Free Trade Zone Shanghai 201210 (CN)
(72) Inventor: YAO, Sheng, Shanghai 201210 (CN); FENG, Hui, Shanghai 201210 (CN); WU, Hai, Shanghai 201210 (CN)
(74) Representative: V.O.
(86) International application number: PCT/CN2021/075481
(87) International publication number: WO 2021/155840

(57) **Abstract**

The present invention relates to use of an anti-PD-1 antibody and/or an antigen-binding fragment thereof in the treatment of a malignancy. In particular, the present invention relates to use of an anti-PD-1 antibody and/or an antigen-binding fragment thereof in the treatment of a sarcoma, preferably alveolar soft part sarcoma, angiosarcoma and undifferentiated polymorphic sarcoma, and use of an anti-PD-1 antibody and/or an antigen-binding fragment thereof in the treatment of lymphoma.

## Description

### TECHNICAL FIELD

The present invention relates to use of an anti-PD-1 antibody or an antigen-binding fragment thereof in the treatment of a malignancy. In particular, the present invention relates to use of an anti-PD-1 antibody or an antigen-binding fragment thereof in the treatment of sarcoma, lung cancer or lymphoma, particularly in the treatment of soft tissue sarcoma, and preferably in the treatment of alveolar soft part sarcoma.

### BACKGROUND

Immune escape is one of the characteristics of cancer. Ahmadzadeh, M. et al disclosed in Blood, 114: 1537-44 that tumor-specific T lymphocytes are often present in the tumor microenvironment, draining lymph nodes and peripheral blood, but are generally unable to control tumor progression due to the network of immunosuppressive mechanisms present in the tumor microenvironment. CD8⁺ tumor infiltrating T lymphocytes (TILs) generally express activation-induced inhibitory receptors, including CTLA-4 and PD-1, while tumor cells often express immunosuppressive ligands, including PD-1 ligand 1 (PD-L1, also called B7-H1 or CD274), which inhibits activation and effector functions of T cells. In the inhibitory mechanism, PD-1 and its ligands have become an important pathway for tumor cells to suppress activated T cells in the tumor microenvironment.

Programmed death receptor 1 (PD-1) plays an important role in immune regulation and maintenance of peripheral tolerance. PD-1 is expressed primarily in activated T and B cells and functions to inhibit the activation of lymphocytes, which is a normal peripheral tissue tolerance mechanism of the immune system that prevents over-reactive immunity. However, the activated T cells infiltrated in the tumor microenvironment highly express PD-1 molecules, and inflammatory factors secreted by the activated leukocytes can induce the tumor cells to highly express ligands PD-L1 and PD-L2 of PD-1, resulting in the continuous activation of the PD-1 pathway of the activated T cells in the tumor microenvironment, and the suppression of T cell function to kill tumor cells. Therapeutic PD-1 antibodies can block this pathway, partially restore the function of T cells, and enable the activated T cells to continuously kill tumor cells.

Blocking the PD-1/PD-L1 pathway has proven to be an effective way to induce a durable anti-tumor response in various cancer indications over the last decade. Monoclonal antibodies (mAbs) blocking the PD/PD-L1 pathway can enhance activation and effector functions of tumor specific T cells, reduce tumor burden, and improve survival rate. Since 2014, the FDA has approved 2 anti-PD-1 monoclonal antibodies (nivolumab and pembrolizumab) and 3 anti-PD-L1 monoclonal antibodies (atezolizumab, avelumab and durvalumab) for the treatment of human tumors, and NMPA has also approved 6 anti-PD-1 antibodies for the treatment of human tumors. There remains, however, a great need for the treatment of sarcoma, particularly alveolar soft part sarcoma, angiosarcoma and undifferentiated polymorphic sarcoma.

### SUMMARY

In one aspect, the present invention provides a method for treating a patient with a malignancy, which comprises administering to the patient a therapeutically effective amount of an anti-PD-1 antibody or an antigen-binding fragment thereof.

In a second aspect, the present invention provides use of an anti-PD-1 antibody and/or an antigen-binding fragment thereof in the preparation of a medicament for treating a patient with a malignancy.

In a third aspect, the present invention provides a kit for treating an individual with a malignancy, which comprises: (a) an anti-PD-1 antibody or an antigen-binding fragment thereof that specifically binds to and inhibits PD-1; and (b) instructions for using the anti-PD-1 antibody or the antigen-binding fragment thereof to treat the malignancy in the individual.

In one or more embodiments of the use, method or kit described herein, the malignancy is selected from sarcoma, lung cancer and lymphoma.

In one or more embodiments of the use, method or kit described herein, the sarcoma is selected from soft tissue sarcoma, angiosarcoma and undifferentiated polymorphic sarcoma (UPS). In a preferred embodiment, the soft tissue sarcoma is alveolar soft part sarcoma (ASPS).

In one or more embodiments, the lymphoma is Hodgkin lymphoma or non-Hodgkin lymphoma. In one or more embodiments of the use, method or kit described herein, the lymphoma patient immunohistochemically tests positive for PD-L1; in one or more embodiments, the lymphoma patient immunohistochemically tests positive for CD8 T cells. In a preferred embodiment, the lymphoma patient immunohistochemically tests positive for PD-L1 and CD8 T cells.

In one or more embodiments of the use, method or kit described herein, the solid tumor patient immunohistochemically tests positive for PD-L1; in one or more embodiments, the solid tumor patient immunohistochemically tests positive for CD8 T cells. In a preferred embodiment, the solid tumor patient immunohistochemically tests positive for PD-L1 and CD8 T cells.

In one or more embodiments of the use, method or kit described herein, the soft tissue sarcoma patient immunohistochemically tests positive for PD-L1; in one or more embodiments, the soft tissue sarcoma patient immunohistochemically tests positive for CD8 T cells. In a preferred embodiment, the soft tissue sarcoma patient immunohistochemically tests positive for PD-L1 and CD8 T cells.

In one or more embodiments of the use, method or kit described herein, the angiosarcoma patient immunohistochemically tests positive for PD-L1; in one or more embodiments, the angiosarcoma patient immunohistochemically tests positive for CD8 T cells. In a preferred embodiment, the angiosarcoma patient immunohistochemically tests positive for PD-L1 and CD8 T cells.

In one or more embodiments of the use, method or kit described herein, the undifferentiated polymorphic sarcoma patient immunohistochemically tests positive for PD-L1; in one or more embodiments, the undifferentiated polymorphic sarcoma patient immunohistochemically tests positive for CD8 T cells. In a preferred embodiment, the undifferentiated polymorphic sarcoma patient immunohistochemically tests positive for PD-L1 and CD8 T cells.

In one or more preferred embodiments of the use, method or kit described herein, the alveolar soft part sarcoma patient immunohistochemically tests positive for PD-L1; in one or more preferred embodiments, the alveolar soft part sarcoma patient immunohistochemically tests positive for CD8 T cells. In a preferred embodiment, the alveolar soft part sarcoma patient immunohistochemically tests positive for PD-L1 and CD8 T cells.

In certain embodiments of the use, method or kit described herein, the individual has received a previous therapy. In certain embodiments, the individual has received a standard therapy. In certain embodiments, the individual has no history of autoimmune diseases; in certain embodiments, the individual is not diagnosed with immunodeficiency; in certain embodiments, the individual has not received systemic therapy for the past 4 weeks; in certain embodiments, the individual has not been treated with systemic steroid drugs or tyrosine kinase inhibitors for the past 2 weeks; in certain embodiments, the individual has no other known progressive malignancies/active brain metastases/cancerous meningitis; in certain embodiments, the individual has not received immune checkpoint blockade therapy.

In one or more embodiments of the use, method or kit described herein, in the administration of the anti-PD-1 antibody for treatment, the anti-PD-1 antibody or the antigen-binding fragment thereof is administered at a therapeutically effective dose of 0. 1 to 10.0 mg/kg, preferably 1 to 10 mg/kg, or of a fixed dose of 80-480 mg, preferably a fixed dose of 240 mg, once every 2-9 weeks, preferably 2-6 weeks. In one or more embodiments, in the administration of the anti-PD-1 antibody for treatment, the anti-PD-1 antibody or the antigen-binding fragment thereof is administered at a therapeutically effective dose of a fixed dose of 80 mg, 240 mg or 480 mg, once every 3-9 weeks. In some preferred embodiments, the anti-PD-1 antibody or the antigen-binding fragment thereof is administered at 1 mg/kg, 3 mg/kg or 10 mg/kg, or at a fixed dose of 240 mg, once every 2-6 weeks. Preferably, the route of administration is intravenous injection.

In the use, method or kit described herein, the individual is a human.

In the use, the method or the kit described herein, by way of a preferred embodiment, the malignancy is sarcoma, lung cancer or lymphoma. By way of a further preferred embodiment, the malignancy is sarcoma. By way of a further preferred embodiment, the malignancy is selected from soft tissue sarcoma, angiosarcoma and undifferentiated polymorphic sarcoma. In a further preferred embodiment, the soft tissue sarcoma is alveolar soft part sarcoma. By way of a further preferred embodiment, the malignancy is lymphoma. By way of a further preferred embodiment, the lymphoma is Hodgkin lymphoma or non-Hodgkin lymphoma. By way of a further preferred embodiment, the lung cancer is non-small cell lung cancer.

In the use, method and kit described herein, the anti-PD-1 antibody is a monoclonal antibody or an antigen-binding fragment thereof. In certain embodiments, the anti-PD-1 antibody specifically binds to PD-1 and blocks the binding of PD-L1 and/or PD-L2 to PD-1. In certain embodiments, the anti-PD-1 antibody specifically binds to PD-L1 or/and PD-L2 and blocks the binding of PD-L1 and/or PD-L2 to PD-1.

In one or more embodiments, the anti-PD-1 antibody or the antigen-binding fragment thereof comprises at least one complementarity determining region (CDR), wherein the CDR has an amino acid sequence selected from SEQ ID NOs: 1, 2, 3, 4, 5 and 6.

In one or more embodiments, the anti-PD-1 antibody or the antigen-binding fragment thereof comprises light chain complementarity determining regions (LCDRs), wherein the LCDRs comprise amino acid sequences set forth in SEQ ID NOs: 1, 2 and 3.

In one or more embodiments, the anti-PD-1 antibody or the antigen-binding fragment thereof comprises heavy chain complementarity determining regions (HCDRs), wherein the HCDRs comprise amino acid sequences set forth in SEQ ID NOs: 4, 5 and 6.

In one or more embodiments, the anti-PD-1 antibody or the antigen-binding fragment thereof comprises light chain complementarity determining regions (LCDRs) and heavy chain complementarity determining regions (HCDRs), wherein the LCDRs comprise amino acid sequences set forth in SEQ ID NOs: 1, 2 and 3, and the HCDRs comprise amino acid sequences set forth in SEQ ID NOs: 4, 5 and 6.

In one or more embodiments, the anti-PD-1 antibody comprises a light chain variable region (VL) and/or a heavy chain variable region (VH), wherein the VL comprises an amino acid sequence set forth in SEQ ID NO: 7, and the VH comprises an amino acid sequence set forth in SEQ ID NO: 8.

In one or more embodiments, the anti-PD-1 antibody is an anti-PD-1 antibody comprising a light chain and/or a heavy chain, wherein the light chain comprises or consists of an amino acid sequence set forth in SEQ ID NO: 9, and the heavy chain comprises or consists of an amino acid sequence set forth in SEQ ID NO: 10.

In one or more embodiments, the anti-PD-1 antibody is selected from nivolumab, pembrolizumab, toripalimab, sintilimab, camrelizumab, tislelizumab and cemiplimab or combinations thereof; preferably, the antibody is toripalimab.

In one or more embodiments, the anti-PD-1 antibody is a monoclonal antibody or an antigen-binding fragment thereof.

In one or more embodiments of the use, method or kit described herein, the anti-PD-1 antibody is toripalimab or an antigen-binding fragment thereof; the tumor is sarcoma, or is lung cancer (including non-small cell lung cancer), or is lymphoma (including Hodgkin lymphoma or non-Hodgkin lymphoma). Preferably, the tumor is soft tissue sarcoma (such as alveolar soft part sarcoma), or is angiosarcoma, or is undifferentiated polymorphic sarcoma. Preferably, the immunohistochemistry test results of the tumor (for PD-L1 and/or CD8 T cells) are as described in any one of the foregoing embodiments.

In one or more embodiments of the use or method described herein, the anti-PD-1 antibody or the antigen-binding fragment thereof is administered at a single dose of about 0.1 mg/kg to about 10.0 mg/kg body weight, e.g., about 0.1 mg/kg body weight, about 0.3 mg/kg body weight, about 1 mg/kg body weight, about 2 mg/kg body weight, about 3 mg/kg body weight, about 5 mg/kg body weight or 10 mg/kg body weight, or selected from a fixed dose of about 80 mg to about 480 mg, e.g., a fixed dose of 80 mg, 120 mg, 240 mg, 360 mg or 480 mg.

In one or more embodiments of the use or method described herein, the anti-PD-1 antibody or the antigen-binding fragment thereof is administered at a frequency of about once every week, once every two weeks, once every three weeks, once every four weeks or once a month, preferably once every two weeks or once every three weeks.

In one or more embodiments of the use or method described herein, the anti-PD-1 antibody or the antigen-binding fragment thereof is administered at a single dose of 1 mg/kg body weight, 3 mg/kg body weight, 10 mg/kg body weight, or of a fixed dose of 80 mg, 240 mg or 480 mg, once every two weeks; or at a single dose of a fixed dose of 240 mg, once every three weeks.

In one or more embodiments of the use or method described herein, the anti-PD-1 antibody or the antigen-binding fragment thereof is administered parenterally, e.g., by intravenous infusion, in a liquid dosage form, e.g., an injection.

In one or more embodiments of the use or method described herein, the anti-PD-1 antibody or the antigen-binding fragment thereof can be administered in cycles of one week, two weeks, three weeks, four weeks, one month, six weeks, two months, three months, four months, five months, half a year or longer; optionally, the cycles each can be identical or different, and at identical or different intervals.

In a fourth aspect, the present invention provides a method for predicting the therapeutic effect of an anti-PD-1 antibody on a tumor patient, which comprises testing the patient for the presence of the fusion gene *ASPSCR1-TFE3,* wherein the presence of the fusion gene *ASPSCR1-TFE3* represents that the tumor patient is suitable for being treated with the anti-PD-1 antibody.

In one or more embodiments, the tumor patient is a solid tumor patient. In one or more preferred embodiments, the tumor patient is a soft tissue sarcoma, angiosarcoma or undifferentiated polymorphic sarcoma patient. In one or more specific embodiments, the tumor patient is a alveolar soft part sarcoma patient.

### BRIEF DESCRIPTION OF THE DRAWINGS

FIG. 1: maximum changes in tumor burden from baseline (n = 22), as evaluated according to RECIST v1.1; (A) lymphoma (n = 11)/IWG 2007; (B) the length of the bars represents the maximum decrease or minimum increase in the target lesions; the unconfirmed partial responses are classified as stable diseases (C) the time of drug exposure, clinical responses and clinical events of the individual patient; wherein, solid tumor burden:sum of target lesions' diameters (RECISTv1.1 criteria); lymphoma tumor burden:sum of target lesions' diameters squared (IWG2007 criteria).
FIG. 2: RECIST v1.1 duration of response to solid tumor (n = 5) and IWG 2007 duration of response to lymphoma (n = 10).
FIG. 3: (A) progression-free survivals of solid tumor patients (n = 22), as determined according to RECIST v1.1 criteria, and progression-free survivals of lymphoma patients (n = 11), as determined according to IWG 2007 criteria; (B) progression-free survivals of sarcoma and non-small cell lung cancer patients, as determined according to RECIST v1.1.
FIG. 4: (A) the overall survivals of solid tumor patients (22 cases) and lymphoma patients (11 cases); (B) the overall survivals of sarcoma and non-small cell lung cancer patients.
FIG. 5: phase I clinical study of toripalimab in advanced or recurrent malignancy patients.
FIG. 6: receptor occupancy (%)-time curves on CD3⁺CD45RA⁻ T cells and CD3⁺CD8⁻CD45RA⁻ T cells after intravenous drip infusion of different doses of toripalimab.
FIG. 7: receptor regulation-time curves on CD3⁺CD45RA⁻ and CD3⁺CD8⁻CD45RA⁻ cells after intravenous drip infusion of different doses of toripalimab.

### DETAILED DESCRIPTION

The present invention relates to a method for treating a malignancy. The method of the present invention comprises administering to a patient in need an anti-PD-1 antibody or an antigen-binding fragment thereof. The malignancy described herein comprises alveolar soft part sarcoma, angiosarcoma, undifferentiated polymorphic sarcoma and lymphoma.

### Terminology

In order to facilitate the understanding of the present invention, some technical and scientific terms are specifically defined below. Unless otherwise specifically defined herein, all technical and scientific terms used herein have the same meaning as commonly understood by those of ordinary skill in the art to which the present invention belongs.

"Administering", "giving" and "treating" refers to introducing a composition comprising a therapeutic agent into a subject using any one of a variety of methods or delivery systems known to those skilled in the art. Routes of administration of the anti-PD-1 antibody include intravenous, intramuscular, subcutaneous, intraperitoneal, spinal or other parenteral routes of administration, such as injection or infusion. "Parenteral administration" refers to modes of administration apart from enteral or local administration, typically by injection, including but not limited to, intravenous, intramuscular, intra-arterial, intrathecal, intralymphatic, intralesional, intracapsular, intraorbital, intracardiac, intradermal, intraperitoneal, transtracheal, subcutaneous, subcuticular, intra-articular, subcapsular, subarachnoid, intraspinal, epidural and intrasternal injection and infusion, and *in vivo* electroporation.

An "adverse event" (AE) described herein is any adverse and often unintended or undesirable sign, symptom, or disease associated with the use of medical treatment. For example, an adverse event may be associated with the activation of the immune system or the amplification of immune system cells in response to treatment. The medical treatment may have one or more related AEs, and the AEs each may have identical or different severity levels.

The term "subject" includes any organism, preferably an animal, more preferably a mammal (such as rat, mouse, dog, cat and rabbit), and most preferably a human. The terms "subject" and "patient" are used interchangeably herein.

An "antibody" described herein refers to any form of antibody that achieves a desired biological or binding activity. Therefore, it is used in the broadest sense, but is not limited to, monoclonal antibodies, polyclonal antibodies, multispecific antibodies, humanized full-length human antibodies, chimeric antibodies and camelized single-domain antibodies. It specifically binds to an antigen and comprise at least two heavy (H) and two light (L) chains interconnected by disulfide bonds, or antigen-binding fragments thereof. Each heavy chain comprises a heavy chain variable region (VH) and a heavy chain constant region comprising three constant domains CH1, CH2 and CH3. Each light chain comprises a light chain variable region (VL) and a light chain constant region comprising one constant domain CL. The VH and VL regions can be further divided into hypervariable regions termed complementarity determining regions (CDRs), which are scattered over more conserved regions termed framework regions (FRs). Generally, both light and heavy chain variable domains comprise FR1, CDR1, FR2, CDR2, FR3, CDR3 and FR4 from N-terminus to C-terminus. Amino acids are typically assigned to each domain according to the following definitions: Sequences of Proteins of Immunological Interest, Kabat et al.; National Institutes of Health, Bethesda, Md.; 5th edition; NIH publication No. 91-3242 (1991): Kabat (1978) Adv. Prot. Chem. 32:1-75; Kabat et al., (1977) J. Biol. Chem. 252:6609-6616; Chothia et al., (1987) J Mol. Biol. 196:901-917 or Chothia et al., (1989) Nature 341:878-883.

The carboxyl-terminal portion of the heavy chain can define a constant region primarily responsible for effector function. Human light chains are generally classified as κ and λ chains. Human heavy chains are generally classified as µ, δ, γ, α or ε chains, and isotypes of the antibody are defined as IgM, IgD, IgG, IgA and IgE, respectively. IgG subclass is well known to those skilled in the art and includes, but is not limited to, IgG1, IgG2, IgG3 and IgG4.

The term "antibody" includes: naturally occurring and non-naturally occurring Abs; monoclonal and polyclonal Abs; chimeric and humanized Abs; human or non-human Abs; fully synthetic Abs; and single chain Abs. Non-human Abs can be humanized by recombinant methods to reduce their immunogenicity in humans.

Unless otherwise specifically indicated, an "antibody fragment" or "antigen-binding fragment" described herein refers to an antigen-binding fragment of an antibody, i.e., an antibody fragment that retains the ability of a full-length antibody to specifically bind to an antigen, e.g., a fragment that retains one or more CDR regions. Examples of antigen-binding fragments include, but are not limited to, Fab, Fab', F(ab')₂, and Fv fragments; a diabody; a linear antibody; a single-chain antibody molecule; and a nanoantibody and a multispecific antibody formed from fragments of the antibody.

A "chimeric antibody" refers to an antibody and a fragment thereof in which a portion of the heavy and/or light chain is identical with or homologous to corresponding sequences of an antibody derived from a particular species (e.g., human) or belonging to a particular antibody class or subclass, while the remainder of the chain is identical with or homologous to corresponding sequences of an antibody derived from another species (e.g., mouse) or belonging to another antibody class or subclass, so long as they exhibit the desired biological activity.

A "human antibody" refers to an antibody that comprises only human immunoglobulin sequences. A human antibody may contain a murine carbohydrate chain if it is produced in mice, mouse cells, or hybridomas derived from mouse cells. Similarly, "mouse antibody" or "rat antibody" refers to an antibody that comprises only mouse or rat immunoglobulin sequences, respectively.

A "humanized antibody" refers to an antibody form containing sequences from both non-human (e.g., murine) and human antibodies. Such antibodies contain minimal sequences derived from non-human immunoglobulins. Typically, a humanized antibody will comprise substantially all of at least one and typically two variable domains, in which all or substantially all of the hypervariable loops correspond to those of a non-human immunoglobulin and all or substantially all of the FR regions are those of a human immunoglobulin. The humanized antibody optionally also comprises at least one portion of an immunoglobulin constant region (Fc), typically a human immunoglobulin constant region.

The term "cancer" refers to a wide variety of diseases characterized by the uncontrolled growth of abnormal cells in the body. Unregulated cell division, growth division and growth lead to the formation of malignant tumors that invade adjacent tissues and can also metastasize to distal parts of the body through the lymphatic system or the blood flow. Examples of cancer include, but are not limited to, carcinoma, lymphoma, leukemia, blastoma, and sarcoma. More specific examples of cancer include squamous cell cancer, myeloma, small-cell lung cancer, non-small cell lung cancer, glioma, Hodgkin lymphoma, non-Hodgkin lymphoma, acute myeloid leukemia, multiple myeloma, gastrointestinal cancer, renal cancer, ovarian cancer, liver cancer, lymphoblastic leukemia, lymphocytic leukemia, colorectal cancer, endometrial cancer, kidney cancer, prostate cancer, thyroid cancer, melanoma, chondrosarcoma, neuroblastoma, pancreatic cancer, glioblastoma multiforme, cervical cancer, brain cancer, gastric cancer, bladder cancer, hepatoma, breast cancer, colon cancer, and head and neck cancer. Another embodiment of cancer includes melanoma. Another specific example of cancer includes metastatic mucosal melanoma. Cancers described herein include those characterized by increased expression of one of or both of PD-L1 and PD-L2 in the tissue samples tested.

The term "lymphoma" refers to a malignancy derived from the lymphohematopoietic system, which is mainly characterized by indolent lymphadenectasis and hepatosplenomegaly. It may affect all tissues and organs in the body, and is accompanied by fever, night sweats, weight loss, pruritus and other systemic symptoms. There are two categories of lymphomas according to the tumor cells: non-Hodgkin lymphoma (NHL) and Hodgkin lymphoma (HL). Hodgkin lymphoma is pathologically characterized by lymphocytes, eosinophils, plasma cells and specific Reed-Steinberg cells in tumor tissues. According to the pathological types, HL can be the nodular lymphocyte-rich type or the classic type. The latter includes the lymphocyte-predominant type, the nodular sclerosis type, the mixed cellularity type and the lymphocyte-depleted type. The incidence of NHL is much higher than that of HL. NHL is a group of independent diseases that are very heterogeneous. Pathologically, NHLs are mainly lymphocytes, histiocytes or reticulocytes with different degrees of differentiation. According to the natural course of NHL, there are three major clinical types: highly aggressive lymphoma, aggressive lymphoma and indolent lymphoma. According to the different lymphocyte origins, there are B-cell lymphoma, T-cell lymphoma and NK-cell lymphoma.

The term "sarcoma" typically refers to a malignancy derived from mesenchymal tissues (including connective tissues and muscles), which occurs mostly in the skin, under the skin, in the periosteum and at both ends of long bones. The "soft tissue sarcoma" is derived from fat, fascia, muscles, fibers, lymph and blood vessels, each with different histological and biological properties and different local infiltration and hematogenous and lymphatic metastases. Lung metastasis is common. According to the incidence of the disease in different body parts, the body parts can be ordered as follows: lower limbs, trunk, head and neck, and upper limbs. Liposarcoma and fibrosarcoma may also occur in the retroperitoneum. The incidence of the disease is high in middle-aged and old people, and there is no gender difference. Soft tissue sarcoma can occur in any body part. About 75% of the lesions are located in the extremities (most commonly in the thighs). Soft tissue sarcomas are mostly malignant. The tumor type varies from age to age. "Angiosarcoma", also known as malignant vascular endothelioma, is a malignancy arising from vascular endothelial cells or mesenchymal cells differentiated towards vascular endothelial cells. It is rare, and the tumor cells have morphological and functional characteristics of normal endothelial cells to some extent. "Alveolar soft part sarcoma (ASPS)" is a rare soft tissue sarcoma that leads to bad prognosis. Its tissue origin is unclear, but its histological and molecular characteristics are special and the clinical presentation is unique. ASPS often occurs in young people. "Undifferentiated polymorphic sarcoma (UPS)" is a malignancy of indeterminate origin, which occurs in both soft tissues and bones. It was first described in 1961. The primary therapies include pre-operative chemotherapy, post-operative chemotherapy and surgical resection.

The term "immunotherapy" refers to the treatment of a subject with a disease or at risk of infection or disease recurrence by a method that includes inducing, enhancing, suppressing or otherwise modifying an immune response. The "treatment" or "method" of a subject refers to any type of intervention or process performed on the subject, or the administration of an active agent to the subject, with the purpose of reversing, alleviating, ameliorating, slowing or preventing the onset, progression, severity, or recurrence of symptoms, complications or conditions, or biochemical indicators associated with the disease.

A "programmed death receptor-1 (PD-1)" refers to an immunosuppressive receptor belonging to the CD28 family. PD-1 is expressed primarily on previously activated T cells *in vivo* and binds to two ligands, PD-L1 and PD-L2. The term "PD-1" used herein includes human PD-1 (hPD-1), variants, isotypes, and species homologs of hPD-1, and analogs having at least one common epitope with hPD-1.

A "therapeutically effective amount" or "therapeutically effective dose" of a medicament or therapeutic agent is any amount of the medicament that, when used alone or in combination with an additional therapeutic agent, protects a subject from the onset of a disease or promotes the regression of a disease as evidenced by a decrease in the severity of disease symptoms, an increase in the frequency and duration of disease symptom-free phase, or the prevention of injury or disability resulting from the affliction of the disease. The ability of a therapeutic agent to promote the regression of a disease can be assessed using a variety of methods known to those skilled in the art, such as in human subjects during clinical trials, in animal model systems that predict human efficacy, or by determining the activity of the agent in an *in vitro* assay.

A therapeutically effective amount of a medicament includes a "prophylactically effective amount" which is any amount of a medicament that, when administered alone or in combination with an anti-neoplastic agent, inhibits the development or recurrence of cancer to a subject at risk of developing cancer or a subject having cancer recurrence.

A "biotherapeutic agent" refers to a biomolecule, such as an antibody or fusion protein, that blocks ligand/receptor signaling in any biological pathway that supports tumor maintenance and/or growth or inhibits an anti-tumor immune response.

Unless otherwise specifically indicated, "CDR" used herein refers to a complementarity determining region of the immunoglobulin variable region defined using the Kabat numbering system.

A "therapeutic anti-PD-1 monoclonal antibody" refers to an antibody that specifically binds to the mature form of a specific PD-1 expressed on the surface of certain mammalian cells. Mature PD-1 does not have a secretory leader sequence, or leader peptide. The terms "PD-1" and "mature PD-1" are used interchangeably herein and are to be understood as the same molecule unless otherwise specifically defined, or clearly seen from the context.

A therapeutic anti-human PD-1 antibody or anti-hPD-1 antibody described herein refers to a monoclonal antibody that specifically binds to mature human PD-1.

A "framework region" or "FR" described herein refers to the immunoglobulin variable region excluding CDR regions.

An "isolated antibody or antigen-binding fragment thereof" refers to a molecule that is in a purified state, and in this case, is substantially free of other biomolecules, such as nucleic acids, proteins, lipids, carbohydrates, or other materials (such as cell debris or growth medium).

A "patient" or "subject" refers to any single subject in need of a medical procedure or participating in a clinical trial, epidemiological study, or serving as a control, including humans and mammals, such as horses, cows, dogs, or cats.

In the following paragraphs, various aspects of the present invention are further described in detail.

### Anti-PD-1 Antibody

A "PD-1 antibody" refers to any chemical compound or biomolecule that binds to the PD-1 receptor, blocks the binding of PD-L1 expressed on cancer cells to PD-1 expressed on immune cells (T, B and NK cells), and preferably blocks the binding of PD-L2 expressed on cancer cells to PD-1 expressed on immune cells. Alternative nouns or synonyms for PD-1 and its ligands include: PDCD1, PD1, CD279 and SLEB2 for PD-1; PDCD1L1, PDL1, B7-H1, B7H1, B7-4, CD274 and B7-H for PD-L1; and PDCD1L2, PDL2, B7-DC and CD273 for PD-L2. In any of the therapy, medicament and use described herein for treating a human individual, the anti-PD-1 antibody blocks the binding of human PD-L1 to human PD-1, and preferably blocks the binding of both human PD-L1 and PD-L2 to human PD-1. The amino acid sequence of human PD-1 can be found at NCBI locus number: NP_005009. The amino acid sequences of human PD-L1 and PD-L2 can be found at NCBI locus numbers: NP_054862 and NP_079515.

As used herein, unless otherwise indicated or described, when referring to the term "anti-PD-1 antibody", it includes antigen-binding fragments of the antibody.

The anti-PD-1 antibody that can be used in any of the uses, methods and kits described herein includes a monoclonal antibody (mAb) or an antigen-binding fragment thereof that specifically binds to PD-1, and preferably specifically binds to human PD-1. The mAb may be a human antibody, a humanized antibody, or a chimeric antibody, and may include a human constant region. In some embodiments, the constant region is selected from human IgG1, IgG2, IgG3, and IgG4 constant regions, and in preferred embodiments, the constant region is a human IgG4 constant region.

In any one of the embodiments of the use, method and kit described herein, the anti-PD-1 antibody is a monoclonal antibody or an antigen-binding fragment thereof, which comprises: light chain CDRs comprising amino acid sequences set forth in SEQ ID NOs: 1, 2 and 3, and heavy chain CDRs comprising amino acid sequences set forth in SEQ ID NOs: 4, 5 and 6.

In any one of the embodiments of the use, method and kit described herein, the PD-1 antibody is a monoclonal antibody that specifically binds to human PD-1 and comprises: (a) a light chain variable region comprising an amino acid sequence set forth in SEQ ID NO: 7, and (b) a heavy chain variable region comprising an amino acid sequence set forth in SEQ ID NO: 8.

In any one of the embodiments of the use, method and kit described herein, the PD-1 antibody is a monoclonal antibody that specifically binds to human PD-1 and comprises: (a) a light chain comprising an amino acid sequence set forth in SEQ ID NO: 9, and (b) a heavy chain comprising an amino acid sequence set forth in SEQ ID NO: 10.

In any one of the embodiments of the use, method and kit described herein, the PD-1 antibody is a monoclonal antibody or an antigen-binding fragment thereof. Table A below provides the amino acid sequence of an exemplary anti-PD-1 antibody mAb for use in the use, method and kit described herein:

**Table A: Light and heavy chain CDRs (Kabat) of an exemplary anti-human PD-1 antibody**

| | |
|---|---|
| LCDR1 | SEQ ID NO: 1 |
| LCDR2 | SEQ ID NO: 2 |
| LCDR3 | SEQ ID NO: 3 |
| HCDR1 | SEQ ID NO: 4 |
| HCDR2 | SEQ ID NO: 5 |
| HCDR3 | SEQ ID NO: 6 |

An example of anti-PD-1 antibodies that bind to human PD-1 and can be used in the use, method and kit described herein is described in WO2014206107. Human PD-1 mAbs that can be used as anti-PD-1 antibodies in the use, method and kit described herein include any one of the anti-PD-1 antibodies described in WO2014206107, including toripalimab (a humanized IgG4 mAb having the structure described in WHO Drug Information; 32(2):372-373 (2018) and comprising light and heavy chain amino acid sequences set forth in SEQ ID NOs: 9 and 10). In certain embodiments, the anti-PD-1 antibody that can be used in the use, method and kit described herein also includes nivolumab and pembrolizumab that have been approved by FDA.

In certain embodiments, the anti-PD-1 antibody that can be used in the use, method and kit described herein also includes anti-PD-L1 monoclonal antibodies that specifically bind to PD-L1 to block the binding of PD-L1 to PD-1, such as atezolizumab, avelumab and durvalumab.

"PD-L1" expression or "PD-L2" expression described herein refers to any detectable expression level of a specific PD-L protein on the surface of a cell or a specific PD-L mRNA within a cell or tissue. PD-L protein expression can be detected in IHC analysis of tumor tissue sections or by flow cytometry using diagnostic PD-L antibodies. Alternatively, PD-L protein expression of tumor cells can be detected by PET imaging using a binding agent that specifically binds to a desired PD-L target (such as PD-L1 or PD-L2).

Methods for quantifying PD-L1 protein expression in IHC analysis of tumor tissue sections are found in, but are not limited to, Thompson, R. H. et al., PNAS 101(49): 17174-17179 (2004); Taube, J.M. et al., Sci Tranisl Med 4, 127ra37(2012); and Topalian, S.L. et al., New Eng.J.Med. 366(26): 2443-2454 (2012), and the like.

In one method, a simple binary endpoint of positive or negative PD-L1 expression is adopted, where the positive expression is defined by the percentage of tumor cells showing histological evidence of cell surface membrane staining. The case where tumor cells on a tumor tissue section account for at least 1% of the total tumor cells is defined as positive PD-L1 expression.

In another method, PD-L1 expression in the tumor tissue section is quantified in tumor cells as well as in infiltrating immune cells. The percentage of tumor cells and infiltrating immune cells exhibiting membrane staining are quantified individually as < 1%, 1% to 50%, and subsequent 50% to 100%. For tumor cells, the PD-L1 expression is counted as negative if the score is < 1%, and positive if the score is ≥ 1%.

In some embodiments, the expression level of PD-L1 by malignant cells and/or by infiltrating immune cells within the tumor is determined to be "overexpressed" or "elevated" based on comparison with the expression level of PD-L1 by an appropriate control. For example, the protein or mRNA expression level of control PD-L1 can be a quantified level in non-malignant cells of the same type or in sections from matched normal tissue.

The "RECIST 1.1 efficacy criteria" described herein refers to the definition of target injury and non-target injury described in Eisenhauver et al., E.A. et al., Eur. J Cancer 45:228-247 (2009) in the context of the measured background.

The term "ECOG" score standard is an indicator of general health status and tolerance to treatment of patients from their physical strength. ECOG score standard for the physical strength is 0 points, 1 point, 2 points, 3 points, 4 points and 5 points. A score of 0 means that the motility is completely normal and has no difference from the motility before onset of disease. A score of 1 means that the person is free to walk and can engage in light physical activities, including general housework or office work, but not in heavy physical activities.

A "sustained response" refers to a sustained therapeutic effect following cessation of treatment with a therapeutic agent or combination method described herein. In some embodiments, the sustained response has a duration that is at least the same as the duration of treatment or at least 1.5, 2.0, 2.5 or 3 times the duration of the treatment.

A "tissue section" refers to a single portion or piece of a tissue sample, such as a tissue slice cut from a sample of normal tissue or a tumor.

As used herein, "treating" cancer refers to administering a treatment regimen described herein (e.g., a combination method of administering an anti-PD-1 antibody and a VEGFR inhibitor) to a subject with or diagnosed with cancer to achieve at least one positive therapeutic effect (e.g., a decrease in cancer cell number, a decrease in tumor volume, a reduction in the rate of cancer cell infiltration into peripheral organs, or a reduction in the rate of tumor metastasis or tumor growth). Positive therapeutic effects in cancer can be measured in a variety of ways (see W. A. Weber, J. Nucl. Med., 50:1S-10S (2009)). For example, T/C ≤ 42% for tumor growth inhibition is the minimum level of anti-tumor activity according to the NCI criteria. It is considered that T/C (%) = median treated tumor volume/median control tumor volume × 100. In some embodiments, the therapeutic effect achieved by the combination of the present invention is any one of PR, CR, OR, PFS, DFS and OS. PFS (also called "time to tumor progression") refers to the length of time during and after treatment for which cancer does not grow, and includes the amount of time a patient experiences CR or PR and the amount of time a patient experiences SD. DFS refers to the length of time during and after treatment for which a patient remains disease-free. OS refers to an extension of life expectancy compared to an initial or untreated individual or a patient. In some embodiments, the response to the combination of the present invention is any one of PR, CR, PFS, DFS, OR or OS, assessed using RECIST 1.1 efficacy criteria. The treatment regimen of the combination of the present invention effective in treating a cancer patient may vary depending upon a variety of factors such as the disease state, age, weight of the patient and the ability of the method to elicit an anti-cancer response in the subject. Embodiments of the present invention may not achieve an effective positive therapeutic effect in each subject, but should be effective and achieve a positive therapeutic effect in a statistically significant number of subjects.

The terms "mode of administration" and "dosing regimen" are used interchangeably and refer to the dosage and time of use of each therapeutic agent in the combination of the present invention.

A "tumor", when applied to a subject diagnosed with or suspected of having cancer, refers to a malignant or potentially malignant neoplasm or tissue mass of any size, and includes primary tumors and secondary neoplasms. Solid tumors typically do not contain abnormal growth or mass of tissue in cysts or fluid areas. Different types of solid tumors are named for the cell type from which they are formed. Examples of solid tumors are sarcomas, carcinomas and lymphomas. Hematologic cancers typically do not form solid tumors.

"Tumor burden" refers to the total amount of tumor mass distributed throughout the body. Tumor burden refers to the total number of cancer cells or the total size of the tumor throughout the body. Tumor burden can be determined by a variety of methods known in the art, such as measuring the size of a tumor using calipers after the tumor is removed from a subject, or using imaging techniques (e.g., ultrasound, bone scanning, computed tomography (CT), or magnetic resonance imaging (MRI) scanning) when the tumor is *in vivo.*

The term "tumor size" refers to the total size of a tumor, which can be measured as the length and width of the tumor. Tumor size can be determined by a variety of methods known in the art, such as measuring the size of a tumor using calipers after the tumor is removed from a subject, or using imaging techniques (e.g., bone scanning, ultrasound, CT, or MRI scanning) when the tumor is *in vivo.*

The term "immunohistochemistry (IHC)" refers to a method for determining antigens (polypeptides and proteins) in tissue cells by developing chromogenic agents (fluoresceins, enzymes, metal ions, isotopes) that label antibodies through chemical reaction based on the principle that antigens specifically binds to antibodies, and performing localized, qualitative and relatively quantitative studies on those antigens. In some embodiments of the present invention, a tumor tissue sample from a subject is tested for PD-L1 or/and CD8⁺ T cells prior to treatment with an anti-PD-1 antibody by staining the anti-human PD-L1 antibody SP142 from Roche (Cat No: M4422), or the CD8 antibody (clone 4B11, Cat# MCA 1817T). In some embodiments, the presence of tumor cells with membrane staining ≥ 1% are defined as PD-L1 positive.

### Pharmaceutical Composition and Dosage

The therapeutic agent described herein can constitute a pharmaceutical composition, such as a pharmaceutical composition comprising the anti-PD-1 antibody described herein or/and an additional anti-cancer agent other than the anti-PD-1 antibody, and an additional pharmaceutically acceptable carrier. As used herein, a "pharmaceutically acceptable carrier" includes any and all solvents, dispersion media, coatings, antibacterial and antifungal agents, isotonic and absorption delaying agents, and the like that are physiologically compatible. Preferably, the carrier suitable for the composition comprising the anti-PD-1 antibody is suitable for intravenous, intramuscular, subcutaneous, parenteral, spinal, or epidermal administration, such as by injection or infusion, while the carrier suitable for the composition comprising an additional anti-cancer agent is suitable for enteral administration, such as oral administration. The pharmaceutical composition of the present invention can contain one or more pharmaceutically acceptable salts, antioxidants, water, non-aqueous carriers, and/or adjuvants such as preserving agent, wetting agent, emulsifying agent, and dispersing agent.

The dosing regimen is adjusted to provide the optimal desired response, such as the maximum therapeutic response and/or the minimum adverse effect. The dose of the anti-PD-1 antibody, when administered in combination with another anti-cancer agent, can range from about 0.01 mg/kg body weight to about 20 mg/kg body weight, or about 0.1 mg/kg body weight to about 10 mg/kg body weight, preferably about 1 mg/kg body weight to about 10 mg/kg body weight, or can be a fixed dose of 120 mg, 240 mg, 360 mg or 480 mg. For example, the dose can be about 0.1 mg/kg body weight, about 0.3 mg/kg body weight, about 1 mg/kg body weight, about 2 mg/kg body weight, about 3 mg/kg body weight, about 5 mg/kg body weight, or about 10 mg/kg body weight. The dosing regimen is generally designed to achieve an exposure that results in sustained receptor occupancy (RO) based on the typical pharmacokinetics of Ab. A representative dosing regimen may be about once a week, about once every two weeks, about once every three weeks, about once every four weeks, about once a month, or longer. In some embodiments, the anti-PD-1 antibody is administered to an individual about once every two weeks.

### Method of the present invention

The present invention relates to a method for treating a patient with cancer, which comprises administering to the cancer patient a therapeutically effective amount of an anti-PD-1 antibody and optionally an additional anti-cancer agent other than the anti-PD-1 antibody. In certain embodiments, lymphoma patients suitable for being treated by the method of the present invention are preferably those who test positive for PD-L1 in peripheral blood, or those who test positive for CD8 T cells in peripheral blood, or those who test positive for PD-L1 and CD8 T cells in peripheral blood. In certain embodiments, sarcoma patients suitable for being treated by the method of the present invention are preferably those who test positive for PD-L1 in peripheral blood, or those who test positive for CD8 T cells in peripheral blood, or those who test positive for PD-L1 and CD8 T cells in peripheral blood.

In certain embodiments, the present invention provides a method for treating an individual/patient with soft tissue sarcoma. In some embodiments, the present invention comprises a method for treating alveolar soft part sarcoma or an individual with alveolar soft part sarcoma. In some embodiments, the method comprises administering to the individual a therapeutically effective dose of an anti-cancer agent serving as an Ab or an antigen-binding fragment thereof that specifically binds to PD-1 receptor and inhibits PD-1 activity. In some embodiments, the present invention comprises a method for treating angiosarcoma or an individual with angiosarcoma. In some embodiments, the method comprises administering to the individual a therapeutically effective dose of an anti-cancer agent serving as an Ab or an antigen-binding fragment thereof that specifically binds to PD-1 receptor and inhibits PD-1 activity. In some embodiments, the present invention comprises a method for treating undifferentiated polymorphic sarcoma (UPS) or an individual with undifferentiated polymorphic sarcoma (UPS). In some embodiments, the method comprises administering to the individual a therapeutically effective dose of an anti-cancer agent serving as an Ab or an antigen-binding fragment thereof that specifically binds to PD-1 receptor and inhibits PD-1 activity.

### Anti-PD-1 antibody suitable for the method of the present invention

The antibody PD-1 antibody suitable for the method of the present invention has an immunosuppressive effect achieved by binding to PD-1 with high specificity and affinity, blocking the binding of PD-L1/2 to PD-1 and inhibiting PD-1 signal transduction. In any of the therapies disclosed herein, the anti-PD-1 antibody comprises an antigen-binding portion or fragment that binds to the PD-1 receptor and exhibits functional properties similar to an intact Ab in inhibiting ligand binding and upregulating the immune system. In some embodiments, anti-PD-1 antibodies or antigen-binding fragments thereof suitable for the method of the present invention are as described in any one of the foregoing embodiments. In some embodiments, the anti-PD-1 antibody or the antigen-binding fragment thereof is an anti-PD-1 antibody or an antigen-binding fragment thereof that cross-competes for binding to human PD-1 with toripalimab. In other embodiments, the anti-PD-1 antibody or the antigen-binding fragment thereof is a chimeric, humanized or human Ab or an antigen-binding fragment thereof. In certain embodiments for treating a human individual, the Ab is a humanized Ab.

In some embodiments, the anti-PD-1 antibody or the antigen-binding fragment thereof comprises a heavy chain constant region of human IgG1 or IgG4 isotype. In some embodiments, the sequence of the IgG4 heavy chain constant region of the anti-PD-1 antibody or the antigen-binding fragment thereof comprises the S228P mutation that replaces a serine residue in the hinge region with a proline residue that is typically present at the corresponding position of an antibody of IgG1 isotype. In certain embodiments of any of the treatment methods described herein that comprises the administration of an anti-PD-1 antibody, the anti-PD-1 antibody is selected from humanized antibodies 38, 39, 41 and 48 described in WO2014206107; preferably, the anti-PD-1 antibody is toripalimab; preferably, the disease is the sarcoma, lung cancer (including non-small cell lung cancer) or lymphoma (including Hodgkin lymphoma or non-Hodgkin lymphoma) described in any one of the embodiments herein; more preferably, the disease is soft tissue sarcoma (such as alveolar soft part sarcoma), angiosarcoma or undifferentiated polymorphic sarcoma.

The present invention also includes a method for predicting the therapeutic effect of an anti-PD-1 antibody on a tumor patient, which comprises testing the patient for the presence of the gene *ASPSCR1-TFE3* fusion, wherein the presence of the gene *ASPSCRI-TFE3* fusion represents that the tumor patient is suitable for being treated with the anti-PD-1 antibody. Conventional methods can be used to test the patient for the presence of the *ASPSCRI-TFE3* fusion. The tumor patient is a solid tumor patient, preferably a sarcoma patient, e.g., a soft tissue sarcoma, angiosarcoma or undifferentiated polymorphic sarcoma patient, and more preferably an alveolar soft part sarcoma patient.

### Abbreviation

The following abbreviations are used throughout the description and examples of the present invention:
- CDR: Complementarity determining region
- DFS: Disease-free survival
- FR: Framework region
- IgG: Immunoglobulin G
- OR: Overall response
- ORR: Objective response rate
- OS: Overall survival
- PD: Progressive disease
- PFS: Progression-free survival
- PR: Partial response
- CR: Complete response
- SD: Stable disease
- DLT: Dose-limiting toxicity
- MTD: Maximum tolerated dose
- AE: Adverse event
- Q2W: One dose every 2 weeks
- Q3W: One dose every 3 weeks
- IRC: Independent review committee
- TRAE: Treatment-related adverse event
- SAE: Serious adverse event
- UPS: Undifferentiated polymorphic sarcoma
- ASPS: Alveolar soft part sarcoma
- HL: Hodgkin lymphoma
- NSCLC: Non-small cell lung cancer
- HNSCC: Head and neck squamous cell carcinoma
- UC: Urothelial carcinoma
- NPC: Nasopharyngeal carcinoma
- IWG: International working group of clinicians

The present invention is further illustrated by the following examples, which should not be construed as limiting the present invention. The contents of all references cited throughout the present application are explicitly incorporated herein by reference.

### Examples

### Example 1. Clinical Study of Using Anti-PD-1 Antibody Alone to Treat Malignancies

Primary inclusion criteria: eligible subjects must (1) be at least 18-65 years old, (2) have advanced or recurrent malignancies, (3) be refractory to standard systemic therapy, (4) have an ECOG score of 0 or 1, (5) have no history of cancerous meningitis, active tuberculosis, autoimmune disease or persistent infection, (6) have not received any systemic therapy for four weeks, (7) have not received systemic steroid medications, minor surgery or tyrosine kinase inhibitor targeted therapy for two weeks.

Subjects must have an evaluable lesion according to RECIST v1.1 criteria, be not allowed to be treated with an anti-tumor drug at the same time, be not allowed to be treated with or have not been treated with anti-CTLA4, anti-PD-1, anti-PD-L1 or anti-PD-L2 antibodies.

Test drug: the anti-PD-1 antibody toripalimab (WO2014206107).

The dosages of the anti-PD-1 antibody used for this study: 1 mg/kg, 3 mg/kg and 10 mg/kg, i.v., once every two weeks (Q2W).

### Study design:

This is a single-arm, phase I clinical trial (NCT02836834). This study was conducted to evaluate the safety and anti-tumor activity of the anti-PD-1 antibody for the treatment of patients with advanced or recurrent malignancies.

From Aug. 3, 2016 to Jun. 23, 2017, a total of 33 patients with metastatic or recurrent cancer were enrolled in the study, including 12 cases of alveolar soft part sarcoma (ASPS), 11 cases of lymphoma, 7 cases of non-small cell lung cancer (NSCLC), 1 case of head and neck squamous cell carcinoma (HNSCC), 1 case of urothelial carcinoma (UC), and 1 case of nasopharyngeal carcinoma (NPC) (see Table 1 for details). The lymphoma subgroup included 9 cases of Hodgkin Lymphoma (HL) and 2 cases of non-Hodgkin lymphoma.

Part A: the dose escalation group, 9 patients included, 3 for each of the 1 mg/kg, 3 mg/kg, and 10 mg/kg dose groups.

Part B: the dose expansion group, 24 patients included, administered at 1 mg/kg (n = 4), 3 mg/kg (n = 16) and 10 mg/kg (n = 4), once every two weeks. See Table 1 for details. 19 men and 14 women were enrolled, at the average age of 32 (ranging from 21 to 65). The median of patient severe pretreatment was 3 previous systemic therapies. 18 patients (55%) received three or more treatments.

Patients received 3rd-line (average) previous systemic therapy, and 18 patients (55%) received third- or later-line previous therapy.

**Table 1: Demographics of the enrolled subjects**

| Characteristic n (%) | | 1 mg/kg, n=7 | 3 mg/kg, n=19 | 10 mg/kg, n=7 | All patients, n = 33 |
|---|---|---|---|---|---|
| Average age (range) | | 43.0 (28-65) | 32.0 (21-54) | 32.0 (22-62) | 32.0 (21-65) |
| Gender | Male | 5(71) | 10(53) | 4 (57) | 19 (58) |
| | Female | 2 (29) | 9 (47) | 3 (43) | 14 (42) |
| Type of cancer | Alveolar soft part sarcoma (ASPS) | 0 | 10 (53) | 2 (29) | 12 (36) |
| | Lymphoma | 2 (29) | 6 (32) | 3 (43) | 11 (33) |
| | Non-small cell lung cancer (NSCLC) | 3 (43) | 2 (11) | 2 (29) | 7 (21) |
| | Head and neck squamous cell carcinoma (HNSCC) | 1 (14) | 0 | 0 | 1 (3) |
| | Nasopharyngeal carcinoma (NPC) | 0 | 1 (5) | 0 | 1 (3) |
| | Urothelial carcinoma (UC) | 1 (14) | 0 | 0 | 1 (3) |
| ECOG | 0 | 4 (57) | 9 (47) | 2 (29) | 15 (46) |
| | 1 | 3 (43) | 10 (53) | 5 (71) | 18 (55) |
| Tumor stage | I | 0 | 0 | 0 | 0 |
| | II | 1 (14) | 1 (5) | 0 | 2 (6) |
| | III | 2 (29) | 3 (16) | 0 | 5 (15) |
| | IV | 4 (57) | 15 (79) | 6 (86) | 25 (76) |
| | Unknown | 0 | 0 | 1 (14) | 1 (3) |
| Previous therapy line | Surgery | 2 (29) | 9 (48) | 5 (71) | 16 (48) |
| | Radiotherapy | 4 (57) | 7 (37) | 3 (43) | 14 (42) |
| | Systemic therapy | 7 (100) | 17 (90) | 6 (86) | 30 (91) |
| | Systemic therapy first line | 0 | 3 (16) | 0 | 3 (9) |
| | Systemic therapy second line | 2 (29) | 6 (32) | 1 (14) | 9 (27) |
| | Systemic therapy ≥ third line | 5 (71) | 8 (42) | 5 (71) | 18 (55) |

Unless otherwise stated, the data are all n (%).

### 1.1 Safety study

By Nov. 15, 2019, the average number of doses of toripalimab that patients received was 16 (ranging from 3 to 72). Toripalimab was well tolerated, and no dose limiting toxicity (DLT) was observed. All 33 (100%) patients experienced at least one adverse event associated with the treatment (Table 2). The most common adverse events are shown in Table 3. Six patients (18%) experienced grade 3 or higher adverse events, including elevated AST (grade 3), elevated creatine kinase (grade 3), fatigue (grade 3), dyspnea (grade 3), dyskinesia (grade 4) (Table 4). Treatment-related severe adverse events (SAE) occurred in 4 cases (12.1%). There were 2 cases of death, one of which was from grade 2 interstitial lung disease and the other one was from grade 3 dyspnea with lung infections. In 4 patients (12.1%), the administration was permanently discontinued due to adverse events, and in 1 patient (3.0%), the treatment was delayed. No infusion reactions occurred in this study. 12 (36.4%) patients experienced grade 1 or 2 immune-related adverse events (irAE) (see Table 5). It can be seen that toripalimab was well tolerated in patients without DLTs, the maximum dose could reach 10 mg/kg Q2W, and no new safety signal was found.

**Table 2: Treatment-related adverse events (TRAE) in the three dose groups**

| | 1 mg/kg (n=7) | 3 mg/kg (n=19) | 10 mg/kg (n=7) | Total (n = 33) |
|---|---|---|---|---|
| | N (%) | N (%) | N (%) | N (%) |
| All adverse events | 7(100.0) | 19(100.0) | 7(100.0) | 33(100.0) |
| Adverse event, grade 3 | 2(28.6) | 4(21.1) | 0(0.0) | 6(18.2) |
| Treatment discontinued for adverse events | 0 | 3 (15.8) | 1 (14.3) | 4(12.1) |
| Dose deferral for adverse events | 0 | 1 (5.3) | 0 | 1 (3.0) |
| Treatment-related adverse events | 1 (14.3) | 2(10.5) | 1 (14.3) | 4(12.1) |
| Adverse events leading to death | 0 | 2 (10.5) | 0 | 2 (6.1) |

**Table 3. Common (≥ 10%) adverse events related to toripalimab therapy in all subjects (n = 33)**

| Adverse events, n (%) | Dose groups | | | |
|---|---|---|---|---|
| | 1 mg/kg, n=7 | 3 mg/kg, n=19 | 10 mg/kg, n=7 | Total, n = 33 |
| All adverse events | 7 (100) | 19 (100) | 7 (100) | 33 (100) |
| Elevated TSH | 4 (57) | 14 (74) | 5 (71) | 23 (70) |
| Cough | 0 | 8 (42) | 5 (71) | 13 (39) |
| Cold-like symptoms | 2 (29) | 6 (32) | 3 (43) | 11 (33) |
| Fever | 2 (29) | 5 (26) | 3 (43) | 10 (30) |
| Leukopenia | 3 (43) | 7 (37) | 0 | 10 (30) |
| Upper respiratory tract infection | 2 (29) | 4 (21) | 3 (43) | 9 (27) |
| White blood cell-positive urine | 1 (14) | 5 (26) | 2 (29) | 8 (24) |
| Elevated AST | 2 (29) | 3 (16) | 2 (29) | 7 (21) |
| Bacteria-positive urine | 1 (14) | 4 (21) | 2 (29) | 7 (21) |
| Rash | 0 | 5 (26) | 2 (29) | 7 (21) |
| Elevated ALT | 2 (29) | 4 (21) | 0 | 6 (18) |
| Reduced hemoglobin | 0 | 5 (26) | 1 (14) | 6 (18) |
| Elevated white blood cell | 1 (14) | 4 (21) | 1 (14) | 6 (18) |
| Lymphopenia | 1 (14) | 5 (26) | 0 | 6 (18) |
| Reduced TSH | 0 | 4 (21) | 2 (29) | 6 (18) |
| Neutropenia | 1 (14) | 4 (21) | 1 (14) | 6 (18) |
| Fatigue | 0 | 5 (26) | 1 (14) | 6 (18) |
| Weight loss | 2 (29) | 2(11) | 1 (14) | 5 (15) |
| Pain | 1 (14) | 3 (16) | 1 (14) | 5 (15) |
| Pruritus | 1 (14) | 2(11) | 2 (29) | 5 (15) |
| Red blood cell-positive urine | 0 | 3 (16) | 1 (14) | 4 (12) |
| Reduced triiodothyronine | 0 | 2(11) | 2 (29) | 4 (12) |
| Elevated free thyroxine | 0 | 3 (16) | 1 (14) | 4 (12) |
| Dyspnea | 1 (14) | 2(11) | 1 (14) | 4 (12) |
| Nodal tachycardia | 1 (14) | 2(11) | 1 (14) | 4 (12) |

**Table 4. Grade 3 or higher treatment-related adverse events**

| Adverse events, n (%) | Dose groups | | | |
|---|---|---|---|---|
| | 1 mg/kg, n=7 | 3 mg/kg, n=19 | 10 mg/kg, n=7 | Total, n = 33 |
| All adverse events | 2 (29) | 4 (21) | 0 | 6 (18) |
| Elevated AST | 0 | 1 (5) | 0 | 1(3) |
| Elevated CK | 0 | 1 (5) | 0 | 1(3) |
| Fatigue | 0 | 1 (5) | 0 | 1(3) |
| Dyskinesia | 1 (14) | 0 | 0 | 1(3) |
| Dyspnea | 0 | 1 (5) | 0 | 1(3) |
| Hematuresis | 1 (14) | 0 | 0 | 1(3) |

**Table 5. Immune-related adverse events**

| n (%) | All | Grade 1 | Grade 2 | Grade 3 | Grade 4 | Grade 5 |
|---|---|---|---|---|---|---|
| Immune-related adverse events (irAEs) | 12 (36.4) | 11 (33.3) | 1 (3.0) | 0 | 0 | 0 |
| Rash | 3 (9.1) | 3 (9.1) | 0 | 0 | 0 | 0 |
| Pruritus | 3 (9.1) | 3 (9.1) | 0 | 0 | 0 | 0 |
| Hypothyroidism | 3 (9.1) | 3 (9.1) | 0 | 0 | 0 | 0 |
| Hyperthyroidism | 2 (6.1) | 2(6.1) | 0 | 0 | 0 | 0 |
| Interstitial lung disease | 2 (6.1) | 1 (3.0) | 1 (3.0) | 0 | 0 | 0 |
| Hyperglycemia | 1 (3.0) | 1 (3.0) | 0 | 0 | 0 | 0 |

### 1.2 Anti-tumor activity study

By Nov. 15, 2019, 12 patients died, 3 patients were lost to follow-up, 13 patients discontinued the treatment, and 5 patients continued the study. Three CR patients (2 cases of HL and 1 case of ASPS) and 1 stable PR patient (HL) voluntarily discontinued the treatment after 6 months or longer of maintenance of CR or PR.

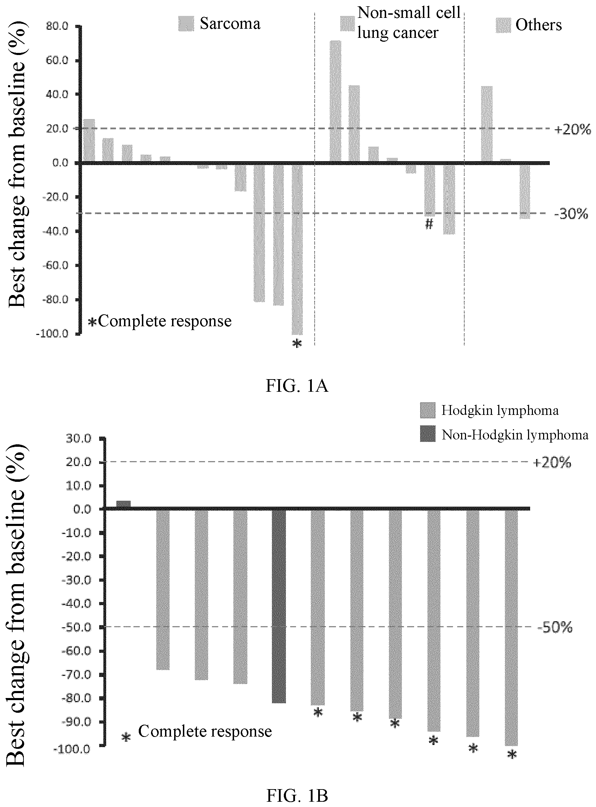

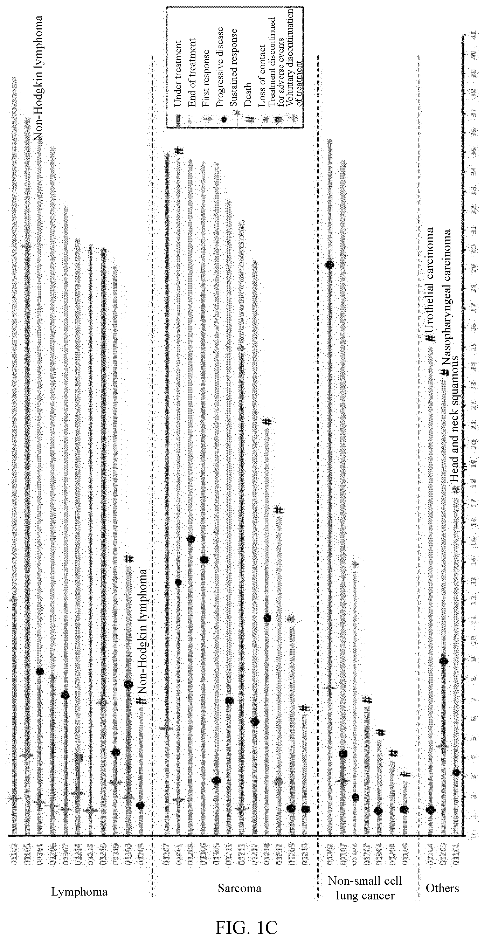

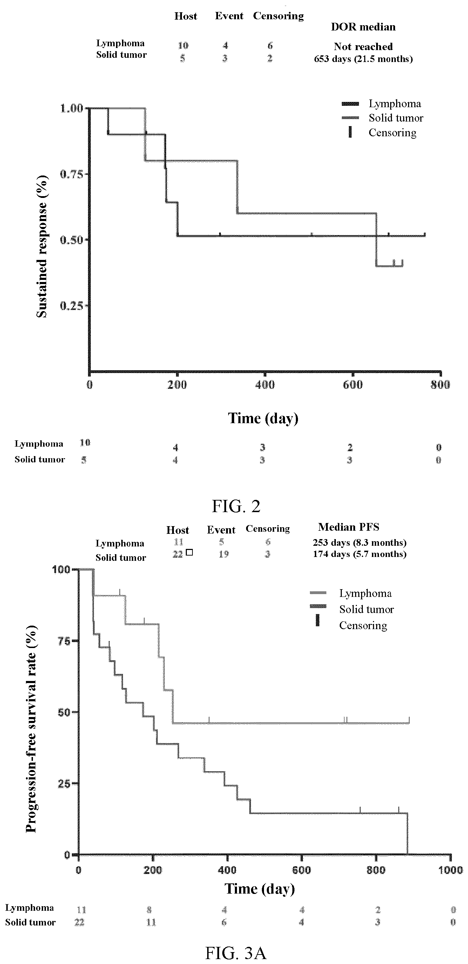

Of the 22 solid tumor patients assessed by RECIST v1.1, 1 achieved a complete response, 4 achieved partial responses, and 13 were in stable condition (see Table 6). The overall ORR for solid tumors was 22.7% (95% CI 7.8-45.4) and the DCR was 81.8% (95% CI 59.7-94.8) (FIG. 1A). In 11 lymphoma patients, both the overall ORR and the DCR were 90.9% (95% CI 58.7-99.8) (FIG. 1B). The median DCR of 5 persons with sustained responses (2 cases of ASPS and 3 cases of HL) was 21.5 months (16.6+, 22.4+, 22.8+, 23.4+ and 25.1+ months, respectively) (FIG. 1C). The median DOR for solid tumors was 21.5 months, while lymphomas did not reach the DOR median (FIG. 2).

**Table 6. Investigators performed a clinical response assessment on solid tumors according to RECIST v1.1 and on lymphoma according to IWG 2007 every 8 weeks**

| Histology | 1 mg/kg (n=7) | 3 mg/kg (n=19) | 10 mg/kg (n=7) | Total (n=33) | ORR, DCR |
|---|---|---|---|---|---|
| ASPS (n=12) | - | 1CR, 2PR, 6SD | 2SD | 1CR, 2PR, 8SD | 25.0%, 91.7% |
| NSCLC (n=7) | 1SD (1uPR) | 2SD | 1PR, 1SD | 1PR, 4SD | 14.2%, 71.4% |
| Lymphoma (n = 11) | 2CR | 3CR, 2PR | 1CR, 2PR | 6CR, 4PR | 91.7%, 91.7% |
| Others (n = 3) | 1SD | 1PR | - | 1PR, 1SD | 33.3%, 66.7% |
| Total | 2CR, 2SD | 4CR, 5PR, 8SD | 1CR, 3PR, 3SD | 7CR, 8PR, 13SD | 45.5%, 84.8% |
| ORR, DCR | 28.6%, 57.1% | 47.4%, 89.5% | 57.1%, 100.0% | 45.5%, 84.8% | |

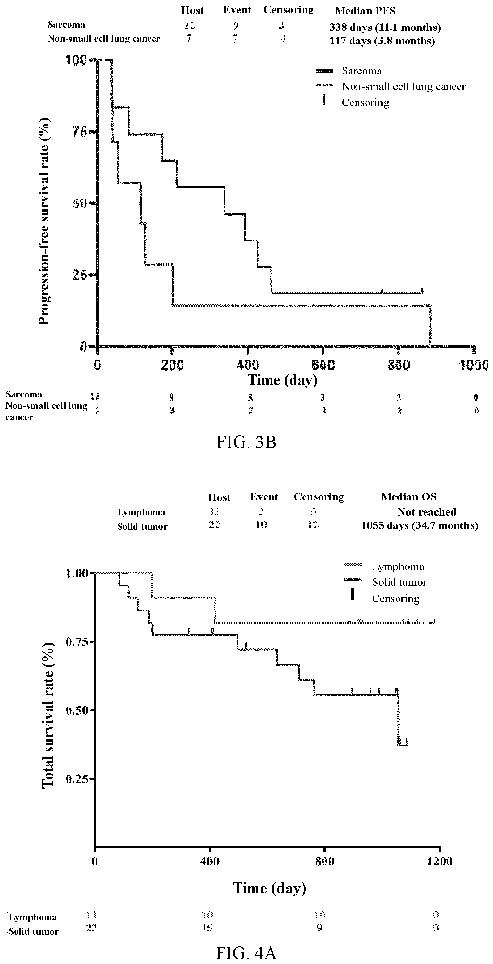

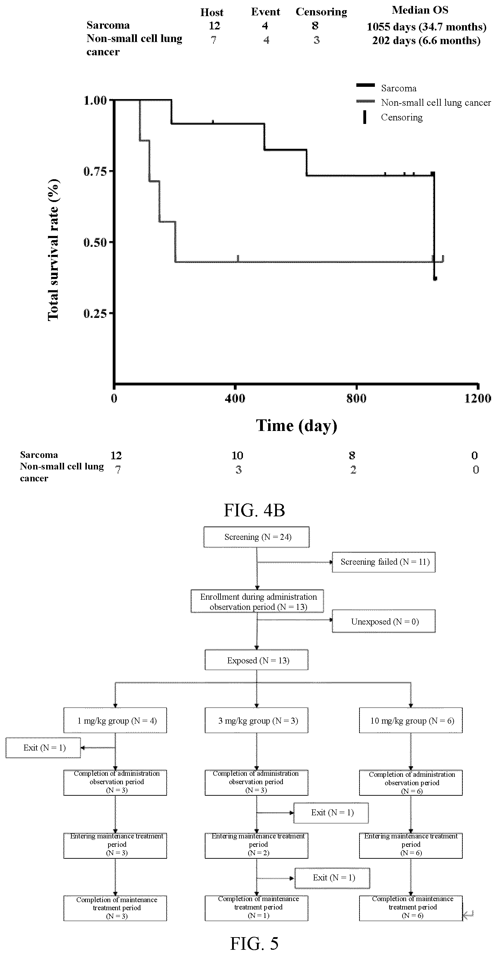

In the 33 patients, the median PFS was 7.1 months (95% CI 4.1-12.9). In 22 cases of solid tumors, the median PFS was 5.7 months. In 11 cases of lymphoma, the median PFS was 8.3 months. The median PFS for ASPS and NSCLC patients were 11.1 and 3.8 months, respectively (FIG. 3). The total number of people and lymphoma subgroups did not reach median OS. The median OS for solid tumors was 34.7 months (95% CI 16.1-NE), while the median OSs for ASPS and NSCLC were 34.7 months and 6.6 months, respectively (FIG. 4). It can be seen that promising and persistent anti-tumor activity was observed in the lymphoma and ASPS expansion groups.

### 1.3 Pharmacokinetics

Toripalimab reached a peak serum concentration (Cₘₐₓ) within 6 hours after the single administration at 1, 3 and 10 mg/kg, i.v. Toripalimab was still detectable on day 56 after the single injections in all three dose groups. The mean serum half-lives for the 1, 3 and 10 mg/kg dose groups were 7.7, 8.0 and 14.2 days, respectively (see Table 7). After 6 consecutive doses, the cumulative fold numbers of toripalimab were 1.7, 1.8 and 2.6, respectively. After injection of multiple-doses of 1 mg/kg, 3 mg/kg and 10 mg/kg, Q2W, the serum half-lives measured were 10.7, 12.1 and 20.1 days, respectively (see Table 8).

**Table 7. Pharmacokinetic profile 56 days after single intravenous injection of toripalimab**

| **PK parameters** | **1 mg/kg (n=3)** | **3 mg/kg (n=3)** | **10 mg/kg (n=3)** |
|---|---|---|---|
| T1/2 (h) | 185±129 | 193±52 | 340±98 |
| Tmax (h) | 0.5-2 | 0.5-6 | 0-2 |
| Cmax (µg/mL) | 17.9±2.6 | 54.0±13.0 | 211.3±32.0 |
| CL (mL/h/kg) | 0.26±0.06 | 0.25±0.06 | 0.13±0.01 |
| Vd (mL/h/kg) | 64.6±31.4 | 65.8±10.3 | 65.5±22.6 |
| AUCO-t (µg·h/mL) | 3781±726 | 12452±2494 | 70244±7242 |
| AUCO-inf (µg·h/mL) | 3944±976 | 12617±2590 | 75806±4373 |
| MRTinf (h) | 325±162 | 284±33 | 499±117 |

**Table 8. Pharmacokinetic profile 56 days after multiple intravenous injections of toripalimab, Q2W**

| **PK parameters** | **1 mg/kg (n=3)** | **3 mg/kg (n=3)** | **10 mg/kg (n=3)** |
|---|---|---|---|
| T1/2 (h) | 256±9 | 290±64 | 482±93 |
| Tmax (h) | 2-6 | 2-48 | 2-24 |
| Cmax (µg/mL) | 25.5±4.5 | 92.3±23.7 | 375.3±98.7 |
| AUCO-t (µg·h/mL) | 4696±926 | 18385±4411 | 87279±19207 |
| AUCO-inf (µg·h/mL) | 8039±1921 | 33619±10484 | 222811±42311 |
| Cmin (µg/mL) | 8.5±2.6 | 34.5±10.0 | 195.8±104.6 |
| CLss (mL/h/kg) | 0.22±0.05 | 0.17±0.05 | 0.12±0.03 |
| Vss (mL/kg) | 80.1±10.0 | 71.5±21.8 | 82.6±31.1 |
| MRTinf_obs (h) | 370±37 | 416±94 | 683±111 |
| Cumulative fold number | 1.67±0.04 | 1.81±0.26 | 2.61±0.39 |

For 1 mg/kg, 3 mg/kg and 10 mg/kg patients, the steady-state concentrations of toripalimab injected in multiple-doses were 8.5±2.6, 34.5±10.0 and 195.8±104.6 µg/mL, respectively. The IC₅₀ for toripalimab blocking was 3 nmol/L (about 0.5 µg/mL), as determined by competitive ELISA analysis. Thus, a 1 mg/kg, Q2W regimen would likely maintain complete occupancy of PD-1 receptors on T cells in peripheral blood. It can be seen that the exposure to toripalimab is characterized by dose-dependence and non-linearity. Toripalimab was injected in multi-doses of 1 mg/kg, 3 mg/kg and 10 mg/kg, and the serum half-lives were 10.7, 12.1 and 20 days, respectively.

### 1.5 Pharmacodynamics

In the three dose groups, toripalimab binds to PD-1 on activated T lymphocytes after the first injection, and full PD-1 receptor occupancy (> 80%) was maintained in most patients throughout the study. Toripalimab had no significant effect on the frequency and activation status of lymphocyte subpopulations.

### 1.6 Immunogenicity

171 ADA samples were obtained from the 33 patients. At different time points after dosing, only 3 samples were obtained from 3 patients, and they tested positive for anti-toripalimab. However, no consecutive ADA positive samples were found, and the clearance of toripalimab was not accelerated in all the 3 ADA positive patients, indicating a lack of neutralizing activity.

### 1.7 Study on correlation between biomarkers and clinical efficacy

Tumor biopsy samples were obtained from 18 patients, among which 4 were cases of Hodgkin lymphoma (HL) and 14 were cases of solid tumors. The samples were tested by immunohistochemical double staining for the expression of PD-L1 and CD8. 4 HL patients tested positive for both PD-L1 and CD8. 57.1% (8/14) of the patients with solid tumors tested positive for PD-L1, and 28.6% (4/14) of the patients tested positive for CD8. All CD8⁺ patients also tested positive for PD-L1. Among the 14 solid tumor patients, the ORR (50% versus 0%) and mPFS (10.8 months versus 3.2 months) of the PD-L1 positive patients were numerically better than that of the PD-L1 negative patients, but there is no statistically significant difference, possibly due to the limited sample size.

The safety and clinical efficacy of PD-1 immune checkpoint inhibitors against alveolar soft part sarcoma was first studied here. 12 alveolar soft part sarcoma patients' chromosomes were examined by fluorescence in situ hybridization (FISH), and it was found that frequent chromosomal translocation occurred between the X chromosome and chromosome 17 of the alveolar soft part sarcoma patients, leading to a new fusion gene ASPSCR1-TFE3. Such translocation represents that alveolar soft part sarcoma could be potentially treated with immune checkpoint site blockers. In this study, toripalimab showed encouraging ORR (25%) and DCR (91.7) for 12 ASPS patients, including 1 CR case and 2 PR cases, and the mPFS and mOS are 11.1 months and 34.7 months, respectively.

Other biomarkers or subgroups of solid tumor patients were analyzed for correlation with clinical efficacy including age, gender, baseline ECOG score and previous routes of treatment. All factors have no significant correlation with clinical efficacy.

### Example 2. Clinical Trial of Using Anti-PD-1 Antibody to Treat Advanced or Recurrent Lymphoma Patients

Primary inclusion criteria: eligible subjects must (1) be 18-70 years old, (2) be histologically or cytopathologically confirmed patients with classic Hodgkin lymphoma or B-cell non-Hodgkin lymphoma, (3) be suffering from recurrence, refractoriness or progression, without available standard therapy at present, (4) have an ECOG score of 0 or 1, (5) have not received systemic chemotherapy for over 4 weeks, have not received radiotherapy for over 4 weeks, have discontinued immunosuppressive doses of systemic medications for at least 4 weeks, and had finished previous anti-tumor biotherapy at least 4 weeks ago, (6) have at least one measurable lesion according to the RECIST v1.1 criteria.

Exclusion criteria: (1) subjects who have a history of other malignancies (excluding cured cervical carcinoma in situ and basal cell carcinoma of the skin), (2) subjects with central nervous system metastases, (3) subjects who have previously received treatment with anti-PD-1 antibodies, anti-PD-L1 antibodies, anti-PD-L2 antibodies or anti-CTLA-4 antibodies, (4) subjects who have previously received CAR-T cell therapy. The distribution screening protocol for subjects is shown in FIG. 5.

Test drug: the anti-PD-1 antibody toripalimab (WO2014206107).

The dosages of the anti-PD-1 antibody used for this study: three dose groups: 1 mg/kg, 3 mg/kg and 10 mg/kg, escalated according to 3+3 principles, administered by intravenous drip infusion once every two weeks (Q2W). The demographic characteristics of the enrolled subjects is shown in Table 9. According to histologic diagnosis, the 1 mg/kg dose group included 2 cases (50.0%) of classic Hodgkin lymphoma and 2 cases (50.0%) of diffuse large B lymphoma; the 3 mg/kg dose group included 1 case (33.3%) of classic Hodgkin lymphoma and 2 cases (66.7%) of diffuse large B lymphoma; the 10 mg/kg dose group included 5 cases (83.3%) of classic Hodgkin lymphoma and 1 case (16.7%) of diffuse large B lymphoma. According to the Ann Arbor staging criteria for lymphoma, the 1 mg/kg dose group included 1 case (25.0%) of stage I, 2 cases (50.0%) of stage II, and 1 case (25.0%) of unknown stage; the 3 mg/kg dose group included 1 case (33.3%) of stage II and 2 cases (66.7%) of stage IV; the 10 mg/kg dose group included 3 cases (50.0%) of stage II and 3 cases (50.0%) of stage IV. The specific pathological examination results are shown in Table 10.

**Table 9: Demographic characteristics of the enrolled subjects**

| Subject No. | Dose group | Age (years) | Gender | Baseline height (cm) | Baseline body weight (kg) | Baseline ECOG score |
|---|---|---|---|---|---|---|
| JS001-01002 | 1mg/kg | 41 | Female | 168 | 75.8 | 0 |
| JS001-01005 | 1mg/kg | 26 | Female | 150 | 45.7 | 1 |
| JS001-01007 | 1mg/kg | 37 | Male | 159 | 55.6 | 1 |
| JS001-01011 | 1mg/kg | 28 | Female | 163 | 49.3 | 1 |
| JS001-01012 | 3mg/kg | 31 | Female | 165 | 67.7 | 0 |
| JS001-01014 | 3mg/kg | 32 | Female | 160 | 50.4 | 1 |
| JS001-01015 | 3mg/kg | 28 | Male | 172 | 64.3 | 0 |
| JS001-01017 | 10mg/kg | 30 | Female | 166 | 57.1 | 0 |
| JS001-01018 | 10mg/kg | 23 | Male | 177 | 101.1 | 0 |
| JS001-01019 | 10mg/kg | 25 | Female | 169 | 56.4 | 1 |
| JS001-01021 | 10mg/kg | 34 | Female | 157 | 66.7 | 0 |
| JS001-01022 | 10mg/kg | 40 | Male | 174 | 75.9 | 0 |
| JS001-01024 | 10mg/kg | 42 | Female | 163 | 56.9 | 0 |

| | | | | | | |
|---|---|---|---|---|---|---|
| Note: the baselines for height and ECOG scores were the height and ECOG scores in the screening period, and the baseline for body weight was the body weight on day 0 of the first dose. | | | | | | |

**Table 10. Pathological test results of the enrolled subjects**

| Indexes | | 1mg/kg(N=4) n(%) | 3mg/kg(N=3) n(%) | 10mg/kg(N=6) n(%) | Total (N = 13) n (%) |
|---|---|---|---|---|---|
| Pathological diagnosis | Classic Hodgkin lymphoma | 2 (50.0) | 1 (33.3) | 5 (83.3) | 8 (61.5) |
| | Diffuse large B lymphoma | 2 (50.0) | 2 (66.7) | 1 (16.7) | 5 (38.5) |
| | Total | 4 (100 ) | 3 (100 ) | 6 (100 ) | 13 (100) |
| Tumor stage | Stage I | 1 (25.0) | 0 (0 ) | 0 (0 ) | 1 (7.7) |
| | Stage II | 2 (50.0) | 1 (33.3) | 3 (50.0) | 6 (46.2) |
| | Stage III | 0 (0 ) | 0 (0 ) | 0 (0 ) | 0 (0 ) |
| | Stage IV | 0 (0 ) | 2 (66.7) | 3 (50.0) | 5 (38.5) |
| | Unknown | 1 (25.0) | 0 (0 ) | 0 (0 ) | 1 (7.7) |
| | Total | 4 (100 ) | 3 (100 ) | 6 (100 ) | 13 (100 ) |

### Study design:

This is a single-arm, single-center, open, single-drug, multiple-dose, dose-escalation phase I clinical trial. This study was conducted to evaluate the safety, tolerability and pharmacokinetics of the anti-PD-1 antibody in patients with advanced lymphoma.

### Dosing regimen

From Sept. 6, 2017 to Dec. 2, 2018, a total of 13 patients with advanced or recurrent lymphoma were included in the study. The subjects were allowed to sequentially enter into 3 dose groups from low to high: 1 mg/kg → 3 mg/kg →- 10 mg/kg. Among the groups, the 1 mg/kg dose group included 3 cases, the 3 mg/kg dose group included 3 cases, and the 10 mg/kg dose group included 6 cases. Administration was performed once by intravenous drip fusion, followed by a 14-day follow-up.

The first administrations to two subjects in the same dose group were at an interval of at least 24 h. The specific interval was determined according to the screening of the subjects.

Each subject was dosed once every 2 weeks, with 6 multiple-doses set in the testing period. After an observation period of 12 weeks, progressive disease or intolerable toxic responses did not occur in the patients. According to the willingness of the patients and the judgment of the investigators, the administration could be continued and maintained, and the subjects entered the maintenance administration period. The administration was maintained until progressive disease or intolerable toxic responses occurred in the patients.

After the second dose was administered to the last subject in the present dose group, a the patient was followed up for at least 24 h. If the dose was safe and well tolerated, the administration of the first dose in the next dose group could be started.

### Trial period:

This trial was a multiple-dose trial with a period of 12 weeks (84 days) in total, including a 12-week period of administration, sample collecting and follow-up.

Subjects received 6 intravenous drip infusions of the test drug according to the regimen, and a safety follow-up was performed on each of days 0, 7, 14, 28, 42, 56, 70 and 84, making a total of 8 follow-ups. Tumor efficacy evaluations were performed on days 0, 42 and 84, making a total of 3 efficacy evaluations (including a baseline phase evaluation).

3 mL of blood was collected from the vein of the elbow that was opposite to the elbow where the infusion was performed at each of the following time points: within 30 min before the first administration (0 hours), on the completion of the first administration, 0.5, 2, 6, 12, 24, 48, 96 and 168 hours after the first administration, on the beginning and completion of each of the second, third, fourth and fifth administrations, within 30 min before the sixth administration (0 hours), on the completion of the sixth administration, and 0.5, 2, 6, 12, 24, 48, 96, 168 and 336 hours after the sixth administration. The serum was separated and cryopreserved at -70 °C to -90 °C. There was a total of 29 time points of blood collection.

In each of the 4 blood collections at the following time points: before the first administration, before the third administration, before the fifth administration, and on day 84 (the last blood collection), additional 3 mL of blood was collected for anti-drug antibody assay. In each of the last blood collection and the blood collections at the following time points: before the first administration, 24 hours after the first administration, and before the second, third and fourth administrations, an additional 3 mL sample of anticoagulated blood was collected for receptor occupancy (RO) assay.

After 12 weeks, the patients who were still taking the drug entered a maintenance treatment period. The collection of the drug safety and efficacy information of the subjects was continued. No more PK and RO assays were performed, but blood samples were still necessarily collected for ADA assay. The administration in the maintenance treatment period was continued until progressive disease or intolerable toxic responses occurred in the patients. After the maintenance administration period began, an adverse event follow-up was performed once every 2 weeks, and a safety observation and an evaluation of the tumor efficacy against tumors were performed once every 6 weeks. In the maintenance treatment period, follow-ups were performed until Dec. 2, 2018, i.e., the last subject was followed up for one year.

### 2.1 Safety and tolerability study

In this study, 1 mg/kg, 3 mg/kg and 10 mg/kg were selected as the doses for the tolerability study. No DLT event occurred in 13 subjects during the experiment, suggesting that toripalimab was well tolerated by the lymphoma patients after several intravenous drip infusions of toripalimab.

13 subjects in this study were graded by NCI-CTCAE toxicity, and no grade 4 and grade 5 TEAEs occurred in the subjects. In the 1 mg/kg dose group, a TEAE graded up to 1 by toxicity occurred in 2 subjects (50.0%), a TEAE graded up to 2 by toxicity occurred in 1 subject (25.0%), and a TEAE graded up to 3 by toxicity occurred in 1 subject (25.0%). In the 3 mg/kg dose group, a TEAE graded up to 1 by toxicity occurred in 1 subject (33.3%), and a TEAE graded up to 3 by toxicity occurred in 2 subjects (66.7%). In the 10 mg/kg dose group, a TEAE graded up to 1 by toxicity occurred in 3 subjects (50.0%), a TEAE graded up to 2 by toxicity occurred in 2 subjects (33.3%), and a TEAE graded up to 3 by toxicity occurred in 1 subject (16.7%).

### 2.2 Anti-tumor activity study

In this study, the drug efficacy in the subjects was evaluated according to the IWG criteria specific to lymphoma and the irRC for immunotherapy. The results show that the three different doses 1 mg/kg, 3 mg/kg and 10 mg/kg all have significant efficacy against lymphoma.

In the optimal efficacy evaluation according to the IWG criteria, in the 1 mg/kg dose group, 2 cases (50.0%) achieved partial responses, 1 case (25.0%) was in stable condition, and 1 case (25.0%) showed progressive disease; in the 3 mg/kg dose group, 2 cases (66.7%) achieved partial responses, and 1 case (33.3%) was in stable condition; in the 10 mg/kg dose group, 4 cases (66.7%) achieved partial responses, and 2 cases (33.3%) were in stable condition. For the 1 mg/kg dose group, the overall response rate was 50.0% [0.068, 0.990], the disease control rate was 75% [0.194, 1.000]. For the 3 mg/kg dose group, the overall response rate was 66.7% [0.094, 1.000], the disease control rate was 100% [0.292, 1.000]. For the 10 mg/kg dose group, the overall response rate was 66.7% [0.223, 1.000], the disease control rate was 100% [0.541, 1.000].

In the optimal efficacy evaluation according to the irRC, in the 1 mg/kg dose group, 1 case (25.0%) achieved a partial response, 1 case (25.0%) was in stable condition, and 2 case (50.0%) were not evaluable; in the 3 mg/kg dose group, 1 case (33.3%) achieved a partial response, and 2 case (66.7%) were not evaluable; in the 10 mg/kg dose group, 4 cases (66.7%) achieved partial responses, and 2 cases (33.3%) were in stable condition. For the 1 mg/kg dose group, the overall response rate was 25.0% [0.006, 0.674], the disease control rate was 50% [0.068, 0.990]. For the 3 mg/kg dose group, the overall response rate was 33.3% [0.008, 0.867], the disease control rate was 33.3% [0.008, 0.867]. For the 10 mg/kg dose group, the overall response rate was 66.7% [0.223, 1.000], the disease control rate was 100% [0.541, 1.000].

### 2.3 Pharmacokinetic study

After multiple consecutive intravenous drip infusions of different doses (1, 3 and 10 mg/kg, administered once every 2 weeks, for a total of 6 administrations) of toripalimab (JS001) into the subjects, the serum drug exposure level increased with the increasing dose. After the first administration, the mean value of t1/2 of each dose group was between 208 h and 429 h; after the last administration, the mean value of t1/2 of each dose group was between 394 h and 434 h. After 6 consecutive administrations, a steady-state level was fundamentally reached, and the accumulation factors of the dose groups were 2.26±0.95, 2.40±0.94 and 2.41±0.39, respectively, indicating that the continuous intravenous infusion of toripalimab led to certain accumulations in the human body. The specific parameters are shown in Tables 11 and 12.

**Table 11. Average plasma concentration after multiple intravenous drip infusions of 1, 3 and 10 mg/kg JS001 into humans**

| (Mean ± SD, µg/mL) | | | | | | |
|---|---|---|---|---|---|---|
| Days Number of administrations | Time (hr) | 1mg/kg JS001 | n | 3mg/kg JS001 | n | 10mg/kg JS001 |
| | | Mean ± SD | | Mean ± SD | | Mean ± SD |
| Day 1 First administration | Before administration | BQL | 4 | BQL | 3 | ^{∗}BQL(BQL,0.01) |
| | On the completion of administration | 19.24±0.97 | 4 | 60.05±7.92 | 3 | 213.10±34.52 |
| | 0.5 | 17.77±2.30 | 4 | 63.82±10.04 | 3 | 209.10±47.34 |
| | 2 | 18.01±0.76 | 4 | 57.73±5.75 | 3 | 221.50±47.27 |
| | 6 | 17.34±0.93 | 4 | 57.87±10.76 | 3 | 197.13±32.65 |
| | 12 | 14.96±0.84 | 4 | 49.98±9.35 | 3 | 188.08±52.72 |
| | 24 | 13.95±2.39 | 4 | 45.59±9.96 | 3 | 170.29±39.9 |
| | 48 | 11.66±1.49 | 4 | 36.96±9.87 | 3 | 144.10±23.11 |
| | 96 | 9.92±1.80 | 4 | 30.88±11.35 | 3 | 121.31±29.20 |
| | 168 | 8.10±1.11 | 3 | 23.19±7.13 | 3 | 101.13±19.88 |
| Day 15 Second administration | Before administration | 4.48±1.14 | 3 | 15.06±5.15 | 3 | 74.94±11.54 |
| | On the completion of administration | 28.31±1.76 | 3 | 77.46±12.02 | 3 | 334.52±48.89 |
| Day 29 Third administration | Before administration | 7.58±1.76 | 3 | 21.85±9.52 | 3 | 118.61±18.01 |
| | On the completion of administration | 28.07±2.25 | 3 | 76.28±13.54 | 3 | 359.97±65.75 |
| Day 43 Fourth administration | Before administration | 8.62±3.00 | 3 | 25.13±21.05 | 3 | 146.70±23.86 |
| | On the completion of administration | 30.20±4.48 | 2 | 74.72±52.23 | 3 | 384.80±89.08 |
| Day 57 Fifth administration | Before administration | 6.74±5.13 | 3 | 26.02±22.52 | 3 | 175.61±36.11 |
| | On the completion of administration | 29.82±6.72 | 3 | 85.27±35.12 | 3 | 415.09±79.79 |
| Day 71 Sixth administration | Before administration | 8.23±3.96 | 3 | 32.90±26.58 | 3 | 233.67±121.20 |
| | On the completion of administration | 25.57±4.42 | 3 | 83.44±29.27 | 3 | 417.32±64.23 |
| | 0.5 | 26.91±2.69 | 3 | 82.26±37.24 | 3 | 414.36±48.28 |
| | 2 | 26.17±1.90 | 3 | 88.77±31.48 | 3 | 414.14±87.37 |
| | 6 | 25.08±5.38 | 3 | 84.44±33.92 | 3 | 404.99±67.83 |
| | 12 | 22.84±4.41 | 3 | 71.91±39.34 | 3 | 383.63±48.87 |
| | 24 | 21.17±3.48 | 3 | 72.79±28.34 | 3 | 380.56±66.10 |
| | 48 | 19.06±4.55 | 3 | 60.92±28.86 | 3 | 334.87±69.31 |
| | 96 | 15.84±4.77 | 3 | 60.14±32.18 | 3 | 284.86±42.65 |
| | 168 | 13.69±4.83 | 3 | 48.73±28.30 | 3 | 280.41±34.14 |
| | 336 | 10.08±5.69 | 3 | 40.09±26.95 | 3 | 209.24±23.73 |

| | | | | | | |
|---|---|---|---|---|---|---|
| Note: ^{∗} indicates a median representation as CV% is greater than 100%. | | | | | | |

**Table 12. Average pharmacokinetic parameters after multiple intravenous drip infusions of 1, 3 and 10 mg/kg JS001**

| PK parameters | Unit | 1mg/kg JS001 | 3mg/kg JS001 | 10mg/kg JS001 |
|---|---|---|---|---|
| | | Mean ± SD(n=3) | Mean ± SD(n=3) | Mean ± SD(n=6) |
| Day 1 | | | | |
| Kel | 1/hr | 0.004±0.001 | 0.003±0 | 0.002±0.001 |
| t1/2 | hr | 208.02±68.92 | 243.42±36.16 | 429.18±270.43 |
| #Tₘₐₓ | hr | 0-6 | 0.50 | 0.00-2.00 |
| Cmax | µg/ml | 19.93±0.17 | 63.82±10.04 | 222.82±46.38 |
| AUC₍₀₋ₜ₎ | hr^{∗}µg/ml | 2983.12±270.79 | 9047.94±2611.11 | 37588.86±7019.66 |
| AUC_{(0-inf)} | hr^{∗}µg/ml | 4400.54±965.5 | 14256.28±4050.73 | 82802.61±22214.47 |
| AUC_{(t-inf)%} | % | 30.63±10.94 | 36.6±4.33 | 51.94±13.45 |
| Vd | ml/kg | 66.98±9.69 | 78.74±29.29 | 71.06±23.93 |
| Cl | ml/hr/kg | 0.24±0.06 | 0.22±0.06 | 0.13±0.03 |
| MRT_{inf} | hr | 290.34±85.03 | 334.08±43.36 | 605.19±376.27 |

| Day 71 | | | | |
|---|---|---|---|---|
| Kel | 1/hr | 0.002±0.001 | 0.002±0.001 | 0.002±0.000 |
| t1/2 | hr | 394.52±229.39 | 428.45±226.28 | 434.29±94.30 |
| #Tₘₐₓ | hr | 0.50-6.00 | 2.00-6.00 | -0.70-6.00 |
| Cmax | µg/mL | 27.30±3.37 | 89.47±32.06 | 444.54±68.64 |
| AUC₍₀₋ₜ₎ | hr^{∗}(µg/mL | 4953.81±1642.89 | 17782.51±9725.92 | 94586.49±12524.34 |
| AUC_{(0-inf)} | hr^{∗}(µg/mL | 11940.25±8404.29 | 48322.54±36651.37 | 226308.30±42737.17 |
| AUC_{(t-inf)%} | % | 48.71±21.98 | 52.72±20.88 | 57.52±6.16 |
| Cmin | µg/ml | 8.23±3.96 | 32.90±26.58 | 193.02±34.60 |
| C_{avg} | µg/ml | 14.72±4.88 | 52.84±28.89 | 281.07±37.23 |
| Fluctuation% | % | 142.13±61.83 | 141.44±94.34 | 89.38±7.35 |
| CLss | ml/hr/kg | 0.22±0.08 | 0.16±0.12 | 0.11±0.01 |
| MRT_{inf} | hr | 543.89±325.93 | 609.74±339.86 | 617.67±111.64 |
| Vz | ml/kg | 108.22±31.01 | 72.74±12.98 | 67.13±16.23 |
| Vss | ml/kg | 102.92±31.91 | 70.98±12.96 | 66.32±14.44 |
| Accumulation_Index | | 2.26±0.95 | 2.40±0.94 | 2.41±0.39 |
| AUC_TAU | hr^{∗}µg/ml | 4947.24±1638.60 | 17755.86±9708.58 | 94438.85±12508.03 |

| | | | | |
|---|---|---|---|---|
| Note: AUC₍₀₋ₜ₎ is AUC₍₀₋₃₃₆₎, and # indicates a range representation. | | | | |

### 2.4 Immunogenicity assay

In this study, the immunogenicity test results show that: after intravenous drip infusion of different doses of toripalimab into subjects, in the 1 mg/kg dose group, ADA was not detected; in the 3 mg/kg dose group, ADA was detected in one subject before the fifth administration and on day 84, and showed an continuous upward trend; in the 10 mg/kg dose group, one subject tested positive before the first administration; as the signal-to-noise ratio was close to the SCP value, it could possibly be a false positive, with a statistical probability of 5%.

### 2.5 Receptor occupancy and immunophenotyping assay

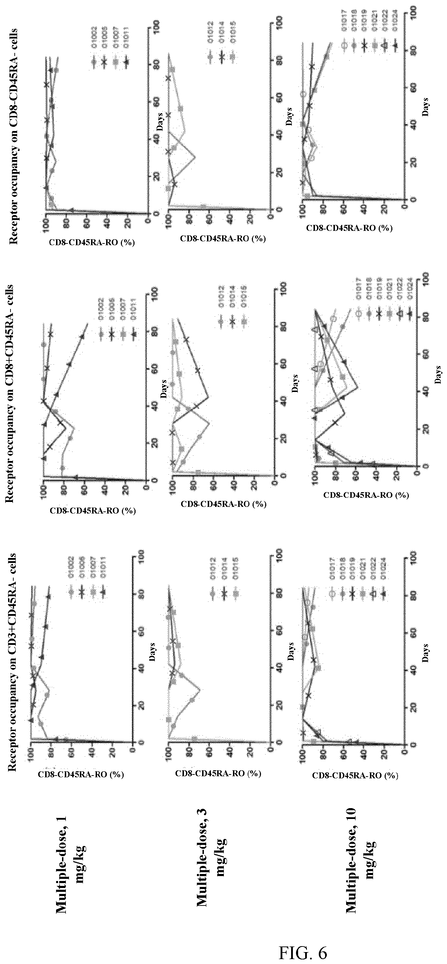

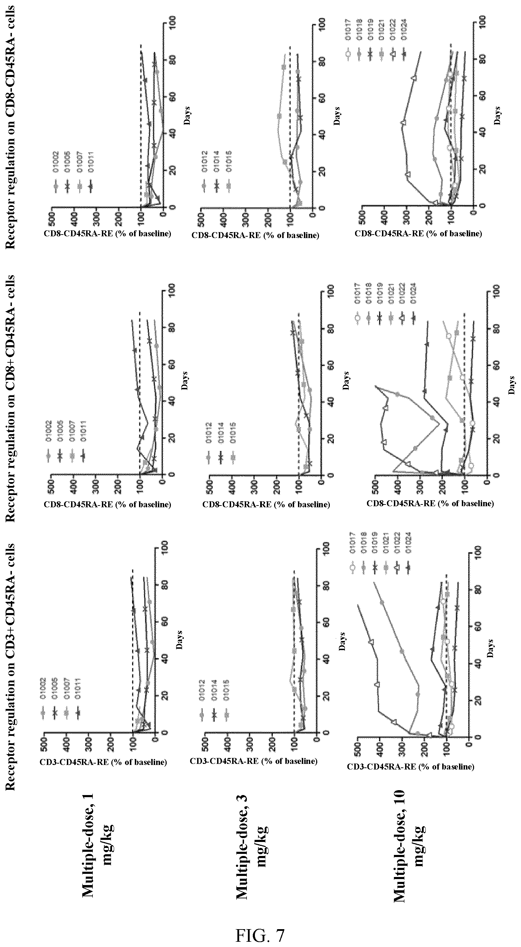

The results of the study in which JS001 was administered to subjects at difference doses (1, 3 and 10 mg/kg, administered once every 2 weeks, for a total of 6 administrations) through multiple consecutive intravenous drip infusions show that: 1. the subjects in the different dose groups could achieve complete occupancy after the first administration of JS001; and during the study, most of the subjects maintained complete receptor occupancy; 2. it was found by flow cytometry analysis of whole blood samples that JS001 had no significant effect on the proportion of T lymphocyte subpopulations. The CD3⁺CD45RA⁻T cell and CD3⁺CD8⁻CD45RA⁻T cell receptor occupancy (%)-time curves after intravenous drip infusion of different doses of toripalimab are shown in FIG. 6. The receptor regulation-time curves on CD3⁺CD45RA⁻ and CD3⁺CD8⁻CD45RA⁻ cells after intravenous drip infusion of different doses of toripalimab are shown in FIG. 7.

### Example 3. Clinical Study of Using Anti-PD-1 Antibody to Treat Sarcoma

### Primary inclusion criteria:

Eligible subjects must (1) be 18 years old or older, (2) have advanced malignancies, (3) be able to be assessed according to RECIST v1.1 and irRECIST criteria, but be difficult to treat with standard therapy or no standard therapy exists, and have an ECOG score of 0 or 1, (4) have normal organ or bone marrow functions, (5) not have certain severe diseases and agree to effective contraception, (6) have not previously received immune therapy, such as, but not limited to, other anti-CTLA-4, anti-PD-1 or anti-L1 antibodies and vaccines.

Test drug: the anti-PD-1 antibody toripalimab (WO2014206107).

### Study design:

This trial was a clinically phase I, multicenter, open study (NCT03474640) in the United States. This study was conducted to evaluate the safety, tolerability, pharmacokinetics, immunogenicity, and anti-tumor activity of toripalimab in patients with advanced malignancies.

The dosages of the anti-PD-1 antibody used for this study: two parts formed in this study: part A, the dose escalation group, administered by intravenous drip infusion, Q2W, in cycles of 28 days; part B, the dose expansion group, administered by intravenous drip infusion, once every 3 weeks (Q3W), in cycles of 42 days.

Part A: a 3+3 design was used and 3-6 subjects were sequentially enrolled in this part at increasing doses of 80 mg, 240 mg and 480 mg. Subjects received the prescribed dose Q2W (±2 days), where no confirmed progressive disease and unacceptable toxicity existed, consent was not withdrawn, no concurrent diseases prevented the use of toripalimab, or where the investigators decided that discontinuing the study and treatment would be most beneficial to the subjects. The dose continued to escalate to a unit dose of 480 mg, or until the MTD or MFD was determined. No more than 18 subjects who had not experienced immunotherapy (had not received immunotherapy before, including but not limited to, other anti-CTLA-4, anti-PD-1 or anti-PD-L1 antibodies, excluding vaccines) might be included in part A.

Part B: 240 subjects with solid tumors including, but not limited to, esophageal cancer, gastric cancer, cholangiocarcinoma, gastrointestinal and pancreatic cancer, sarcoma, soft tissue sarcoma (excluding leiomyosarcoma) and chondrosarcoma, and other tumors including nasopharyngeal carcinoma and liver cancer; patients who had received at least one metastatic treatment were included in the study. Patients with high microsatellite instability (MSI-H)/deficient mismatch repair (dMMR) tumors were eligible for enrollment. The dosage for evaluation was 240 mg, Q3W.

### Anti-tumor activity study

By Sept. 24, 2019, a phase I clinical trial of toripalimab was conducted in the United States, including a total of 90 subjects. Of them, 3 subjects were angiosarcoma patients and 3 subjects were undifferentiated polymorphic sarcoma (UPS) patients. Of these 6 subjects, 2 angiosarcoma subjects achieved partial responses, with an ORR of 67%; 1 undifferentiated polymorphic sarcoma subject achieved a partial response, with an ORR of 33.3%. It can be seen that toripalimab had significant therapeutic effects on angiosarcoma and undifferentiated polymorphic sarcoma.

## Claims

1. Use of an anti-PD-1 antibody and/or an antigen-binding fragment thereof in the preparation of a medicament for treating a patient with a malignancy.

2. The use according to claim 1, wherein the malignancy is selected from sarcoma, lung cancer and lymphoma.

3. The use according to claim 2, wherein the sarcoma is selected from soft tissue sarcoma, angiosarcoma and undifferentiated polymorphic sarcoma.

4. The use according to claim 3, wherein the soft tissue sarcoma is alveolar soft part sarcoma (ASPS).

5. The use according to claim 2, wherein the lymphoma is Hodgkin lymphoma or non-Hodgkin lymphoma.

6. The use according to claim 5, wherein the lymphoma patient immunohistochemically tests positive for PD-L1; or the lymphoma patient immunohistochemically tests positive for CD8 T cells; or the lymphoma patient immunohistochemically tests positive for PD-L1 and CD8 T cells.

7. The use according to claim 2 or 3, wherein the sarcoma patient immunohistochemically tests positive for PD-L1; or the sarcoma patient immunohistochemically tests positive for CD8 T cells; or the sarcoma patient immunohistochemically tests positive for PD-L1 and CD8 T cells.

8. The use according to claim 2, wherein the lung cancer patient immunohistochemically tests positive for PD-L1; or the lung cancer patient immunohistochemically tests positive for CD8 T cells; or the lung cancer patient immunohistochemically tests positive for PD-L1 and CD8 T cells.

9. The use according to any one of claims 1 to 8, wherein the anti-PD-1 antibody comprises a complementarity determining region, wherein the complementarity determining region comprises an amino acid sequence set forth in SEQ ID NO: 1, 2, 3, 4, 5 or 6.

10. The use according to any one of claims 1 to 8, wherein the anti-PD-1 antibody comprises light chain complementarity determining regions (LCDRs) and/or heavy chain complementarity determining regions (HCDRs), wherein the LCDRs comprise amino acid sequences set forth in SEQ ID NOs: 1, 2 and 3, and the HCDRs comprise amino acid sequences set forth in SEQ ID NOs: 4, 5 and 6.

11. The use according to any one of claims 1 to 8, wherein the anti-PD-1 antibody comprises a light chain variable region (VL) and/or a heavy chain variable region (VH), wherein the VL comprises an amino acid sequence set forth in SEQ ID NO: 7, and the VH comprises an amino acid sequence set forth in SEQ ID NO: 8.

12. The use according to any one of claims 1 to 8, wherein the anti-PD-1 antibody is an anti-PD-1 antibody comprising a light chain and/or a heavy chain, wherein the light chain comprises or consists of an amino acid sequence set forth in SEQ ID NO: 9, and the heavy chain comprises or consists of an amino acid sequence set forth in SEQ ID NO: 10.

13. The use according to any one of claims 1 to 8, wherein the anti-PD-1 antibody is selected from one or more of nivolumab, pembrolizumab, toripalimab, sintilimab, camrelizumab, tislelizumab and cemiplimab, and is preferably toripalimab.

14. The use according to any one of claims 1 to 8, wherein the anti-PD-1 antibody is a monoclonal antibody or an antigen-binding fragment thereof.

15. The use according to any one of claims 1 to 14, wherein the anti-PD-1 antibody or the antigen-binding fragment thereof is administered at a single dose of about 0.1 mg/kg to about 10.0 mg/kg body weight, e.g., about 0.1 mg/kg body weight, about 0.3 mg/kg body weight, about 1 mg/kg body weight, about 2 mg/kg body weight, about 3 mg/kg body weight, about 5 mg/kg body weight or 10 mg/kg body weight, or selected from a fixed dose of about 80 mg to about 480 mg, e.g., a fixed dose of 80 mg, 120 mg, 240 mg, 360 mg or 480 mg.

16. The use according to claim 15, wherein the anti-PD-1 antibody or the antigen-binding fragment thereof is administered at a frequency of about once every week, once every two weeks, once every three weeks, once every four weeks or once a month, preferably once every two weeks or once every three weeks.

17. The use according to claim 16, wherein the anti-PD-1 antibody or the antigen-binding fragment thereof is administered at a single dose of 1 mg/kg body weight, 3 mg/kg body weight, 10 mg/kg body weight, or of a fixed dose of 80 mg, 240 mg or 480 mg, once every two weeks; or at a single dose of a fixed dose of 240 mg, once every three weeks.

18. The use according to claim 15, wherein the anti-PD-1 antibody or the antigen-binding fragment thereof is administered parenterally, e.g., by intravenous infusion, in a liquid dosage form, e.g., an injection.

19. The use according to claim 15, wherein the anti-PD-1 antibody or the antigen-binding fragment thereof can be administered in cycles of one week, two weeks, three weeks, four weeks, one month, six weeks, two months, three months, four months, five months, half a year or longer; optionally, the cycles each can be identical or different, and at identical or different intervals.

20. A method for predicting the therapeutic effect of an anti-PD-1 antibody on a tumor patient, comprising testing the patient for the presence of the fusion gene *ASPSCR1-TFE3,* wherein the presence of the fusion gene *ASPSCRI-TFE3* represents that the tumor patient is suitable for being treated with the anti-PD-1 antibody.

21. The method according to claim 20, wherein the tumor patient is a solid tumor patient, preferably a soft tissue sarcoma, angiosarcoma or undifferentiated polymorphic sarcoma patient, and more preferably an alveolar soft part sarcoma patient.
